# EUROPÄISCHE PATENTANMELDUNG

(11) **EP 0 864 651 A2**
(43) Veröffentlichungstag der Anmeldung: **16.09.1998**
(21) Anmeldenummer: 98104597.4
(22) Anmeldetag: 13.03.1998
(51) Int. Cl.: C12N 15/85, A61K 31/70

(54) **Promotor des cdc25B Genes, seine Herstellung und Verwendung**

(30) Priorität: 14.03.1997 DE 19710643
(71) Anmelder: HOECHST AKTIENGESELLSCHAFT, 65929 Frankfurt am Main (DE)
(72) Erfinder: Körner, Kathrin, 35037 Marburg (DE); Müller, Rolf, Prof. Dr., 35037 Marburg (DE); Sedlacek, Hans-Harald, Prof. Dr., 35041 Marburg (DE)
(74) Vertreter: Bösl, Raphael, Dr. rer. nat., Dipl.-Ing.

(57) **Zusammenfassung**

Die Erfindung betrifft den Promotor des cdc25B-Genes, Verfahren zum Auffinden von cdc25B-Promotoren und die Verwendung zur Herstellung eines Arzneimittels.

## Beschreibung

Die Erfindung betrifft den Promotor des cdc 25B-Genes, Verfahren zum Auffinden von cdc 25B-Promotoren und die Verwendung zur Herstellung eines Arzneimittels.

Die Zellteilung wird in die aufeinanderfolgenden Phasen G₀ oder G₁, S, G₂ und M unterteilt. Die S-Phase ist die DNS Synthese-Phase, ihr folgt die Übergangsphase G₂ (G₂-Phase), an die sich die Mitose-Phase (M-Phase) anschließt, in welcher sich die Mutterzelle in zwei Tochterzellen teilt. Zwischen der M-Phase und der S-Phase liegt die Ruhephase G₀ (G₀-Phase) oder die Übergangsphase G₁ (G₁-Phase).

Die Zellteilung wird durch eine Gruppe von Proteinkinasen, den Cyclin/cdk-Komplexen vorangetrieben. Diese bestehen aus einer katalytischen Untereinheit [cyclin dependent kinase (cdk, zum Beispiel cdk-1, -2, -3, -4, -5, -6, -7 oder -8) und einer regulativen Untereinheit, dem Cyclin (zum Beispiel Cyclin A, - B1-B3, - D1-D3, - E, - H, oder - C].

In jeder Phase des Zellzyklus sind unterschiedliche cdk-Komplexe besonders aktiv, so in der mittleren G₁-Phase die cdk-Komplexe cdk4/Cyclin D1-3 und cdk6/Cyclin D1-3, in der späten G₁-Phase der cdk-Komplex cdk2/Cyclin E, in der S-Phase der cdk-Komplex cdk2/Cyclin A sowie in der G₂/M-Übergangsphase die cdk-Komplexe cdk1/Cyclin B1-3 und cdk1/Cyclin A.

Die Aktivität der Cyclin/cdk-Komplexe besteht in der Phosphorylierung und damit Aktivierung oder Inaktivierung von Proteinen, die an der Kontrolle der DNA-Synthese und der Mitose direkt oder indirekt beteiligt sind.

Korrespondierend mit ihrer Funktion im Zellzyklus werden die Gene für einige Cycline und cdk's periodisch transkribiert und/oder periodisch aktiviert oder inhibiert, so durch den regulierten Abbau von Cyclinen, durch zellzyklusphasenspezifische Bindung von Inhibitoren (z.B. p16^{INK4A}, p15^{INK4B}, p21^{Cip1}, P27^{Kip1}, p18^{INK4C}, p19^{INK4D}, P57) oder durch Modifizierung durch aktivierende (z.B. cdc25-Phosphatasen oder cdk7/Cyclin H) oder inhibierende (z.B. wee 1 kinase) Enzyme (Übersicht bei Zwicker und Müller, Progr. Cell Cycle Res., 1, 91 (1995); La Thangue, Curr. Opin. Cell Biol., 6, 443 (1994); MacLachlan et al., Crit. Rev. Eukaryotic Gene Expr., 5, 127 (1995)).

Höhere Eukaryonten besitzen zumindest drei cdc25 Phosphatasen, nämlich cdc25A, cdc25B und cdc25C. Die cDNA der Gene dieser Phosphatasen wurden bereits kloniert und analysiert (Okazaki et al., Gene 178, 111 (1996); Galaktionow et al., Cell 67, 1181 (1991)). Alle drei Phosphatasen treten periodisch im Zellzyklus auf. Die aktivierenden Funktionen dieser cdc25 Phosphatasen sind jedoch offensichtlich unterschiedlich (Jinno et al., EMBO J. 13, 1549 (1994); Honda et al., FEBS Lett. 318, 331 (1993); Hoffmann et al., EMBO J. 13, 4302 (1994)):

cdc25A wird vorwiegend in der späten G₁-Phase exprimiert, regelt besonders den Übergang von der G₁-Phase in die S-Phase (Start des Zellzyklus) durch Aktivierung von cdk/Cyclin-Komplexen und wird durch Myc (Transkription) und Raf (Aktivität) reguliert. cdc25B dephosphoryliert die Tyrosine (Tyrosin-14, -15) in der ATP-Bindungstasche der cdk-1, was zu deren Aktivierung führt, kann unabhängig von cdk-1 durch Cyclin B (1-3) stimuliert werden und seine Expression ist in Virus (SV40 oder HPV) infizierten Zellen dereguliert und verstärkt. cdc25C dephosphoryliert die Tyrosine (Tyrosin-14, -15) in der ATP-Bindungstasche der cdk-1, was zur deren Aktivierung führt, wird besonders in der G₂-Phase exprimiert und regelt den Eintritt in die M-Phase.

Die periodische Expression von cdc25C in der G₂-Phase des Zellzyklus wird im wesentlichen durch ein Element (CDE-CHR) in der Promotorregion von cdc25C geregelt, welches in der G₀-/G₁-Phase von einem reprimierenden Protein besetzt ist und in der G₂-Phase frei ist. Die Nukleotidsequenz dieses Promotorelementes konnte identifiziert und gleichermaßen auch in den Promotoren der Gene für Cyclin A und cdk-1 gefunden werden, während im Promotor für B-myb eine zum Teil unterschiedliche Nukleotidsequenz (E2FBS-CHR) nachgewiesen wurde. Die Untersuchung der zellzyklusabhängigen Funktionsweise dieser Promotorelemente zeigte, daß ihre Blockade in der G₀-/G₁-Phase von einer Hochregulierung der Transkription des jeweiligen Genes gefolgt wird, welche beim B-myb Gen besonders früh (in der mittleren G₁-Phase), beim Cyclin A in der G₁/S-Übergangsphase, beim cdk-1 Gen in der S-Phase und beim cdc25C Gen erst in der späten S-Phase stattfindet (Zwicker und Müller, Progress in Cell Cycle Res. 1, 91 (1995); Lucibello et al., EMBO J. 14, 132 (1995); Liu et al., Nucl. Acids Res. 24, 2905 (1995); Zwicker et al., Nucl. Acids Res. 23, 3822 (1995); EMBO J. 14, 4514 (1995)).

Es wurde des weiteren gefunden, daß das Element CDE-CHR (des Promotors für das Cyclin 25C, Cyclin A und cdk-1 Gen) wie auch das Element E2FBS-CHR (des Promotors für das B-myb Gen) nicht nur die Aktivierung und Transkription der homologen Gene in der G₀-/G₁-Phase inhibieren kann, sondern auch die Aktivierung und Transkription anderer Gene. (siehe z.B. WO96/06943, DE19605274.2, DE19617851.7, WO96/06940, WO96/06938, WO96/06941 und WO96/06939).

In diesen Patentanmeldungen wird offenbart, einen zellzyklusabhängigen Promotor mit einem unspezifischen, zellspezifischen, virusspezifischen oder metabolisch aktivierbaren Promotor zur regulierten Aktivierung der Transkription eines Effektorgenes zu kombinieren, welches ein Protein für die Prophylaxe und/oder Therapie einer Erkrankung kodiert. Derartige Erkrankungen können beispielsweise Tumorerkrankungen, Leukämien, Autoimmunerkrankungen, Arthritiden, Allergien, Entzündungen, Abstoßungen von transplantieren Organen, Erkrankungen des Blutkreislaufsystems, des Blutgerinnungssystems, Infektionen oder Schäden des zentralen Nervensystems sein.

Ein besonderes Beispiel dieser Kombinationsmöglichkeit von unterschiedlichen Promotoren mit einem zellzyklusspezifischen Promotorelement ist der sogenannte chimäre Promotor. In diesem chimären Promotor wird die Aktivität einer unspezifischen, zellspezifischen, virusspezifischen oder metabolisch aktivierbaren Aktivierungssequenz (bzw. Promotorsequenz) durch das sich stromabwärts unmittelbar anschließende Promotorelement CDE-CHR oder E2FBS-CHR weitgehend auf die S- und G₂-Phase des Zellzyklus beschränkt.

Weiterführende Untersuchungen zur Funktionsweise, insbesondere des Promotorelementes CDE-CHR, ergaben, daß die durch das CDE-CHR Element zellzyklusabhängige Regulation einer stromaufwärts gelegenen Aktivatorsequenz weitgehend davon abhängig ist, daß die Aktivierungssequenz von Transkriptionsfaktoren mit glutaminreichen Aktivierungsdomänen aktiviert wird (Zwicker et al., Nucl. Acids Res. 23, 3822 (1995)).

Zu derartigen Transkriptionsfaktoren gehört beispielsweise Sp1 und NF-Y.

Dieses schränkt konsequenterweise die Verwendung des Promotorelementes CDE-CHR für chimäre Promotoren ein. Gleiches ist für das Promotorelement E2F-BS-CHB des B-myb Genes anzunehmen (Zwicker et al., Nucl. Acids Res. 23, 3822 (1995)).

Aufgabe der vorliegenden Erfindung ist daher, zellzyklusspezifische Promotoren und Promotorelemente zu finden, deren G₀- und G₁-spezifische Repression von anderen Bedingungen abhängig ist als von denen, welchen das Promotorelement CDE-CHR unterliegt.

Bei einer Analyse der Nukleotidsequenz des Promotors des murinen cdc25B Genes wie auch der Nukleotidsequenz der sich stromabwärts anschließenden 5'-nicht kodierenden Region einschließlich der Initiations- (bzw. Start-) Region des cdc25B Genes (Nukleotidsequenz -950 bis + 167) wurde gefunden, daß die funktionellen Bereiche der cdc25B Promotorsequenz zwei E-Boxen, zwei (putative) E2F-Bindestellen, vier (putative) SP1-Bindestellen, eine (putative) NF-Y Bindestelle und eine TATA-Box enthalten. Nukleotidsequenzen mit Homologie zur CDE-CHR oder E2FBS-CHR waren überraschenderweise nicht zu finden. Demzufolge sind die funktionellen Bereiche der cdc25B Promotorsequenz eindeutig unterschiedlich von den funktionellen Bereichen des Promotors des cdc25C, des Cyclin A, des cdk1- und des B-myb Genes. Zudem ist überraschend, daß bislang noch kein Promotor eines Zellzyklusgenes beschrieben wurde, der eine funktionelle TATA-Box enthielt.

Ein Gegenstand der vorliegenden Erfindung ist daher der Promoter des cdc25B-Genes, enthaltend eine Sequenz, die mit einer Sequenz gemäß Tabelle 1 (SEQ ID No: 7) oder einem funktionellen Teil davon unter stringenten Bedingungen hybridisiert, insbesondere der Promoter mit der Sequenz gemäß Tabelle 1 (SEQ ID No: 7) oder einem funktionellen Teil davon.

Die gesamte Sequenz oder Fragmente des cdc25B Promotors wurden stromaufwärts eines Luziferasegens in ein Plasmid kloniert und diese Plasmide in ruhende und proliferierende Fibroblasten der Maus und des Menschen transfiziert und die Menge der exprimierten Luziferase gemessen.

Es zeigte sich, daß (im Gegensatz zu proliferierenden Zellen) in ruhenden Zellen die klonierte cdc25B Sequenz (Promotor und 5'-nicht kodierende Region; ca. - 950 bis ca. + 167) zu einer starken Suppression der Expression des Luziferasegens führt, wobei diese Suppression durch Deletionen am 5'-Ende des cdc25B Promotors (von ca. -950 bis ca. + 167 nach ca. -30 bis ca. + 167) schrittweise reduziert wurde.

Deletionsfragmente kleiner als ca. -180 bis ca. + 167 führten zu einer Reduktion der Promotoraktivität auch in proliferierenden Zellen.

Als funktionelle Teile im Sinne der vorliegenden Erfindung versteht man daher insbesondere die oben näher bezeichneten Bindestellen für Trankskriptionsfaktoren, vor allem wenn sie mehr als ca. 50% des gesamten Promotors umfassen. Besonders bevorzugt sind funktionelle Teile, die die TATA-Box, mindestens eine SP1-Bindestelle, mindestens eine NFY-Bindestelle und gegebenenfalls mindestens eine E2F-Bindestelle und gegebenenfalls mindestens eine E-Box enthält, so daß eine zellzyklusabhängige Expression eines Effektorgens erreicht werden kann. Hierzu gehören insbesondere Promotorsequenzen des murinen, aber auch des humanen cdc25B Gens.

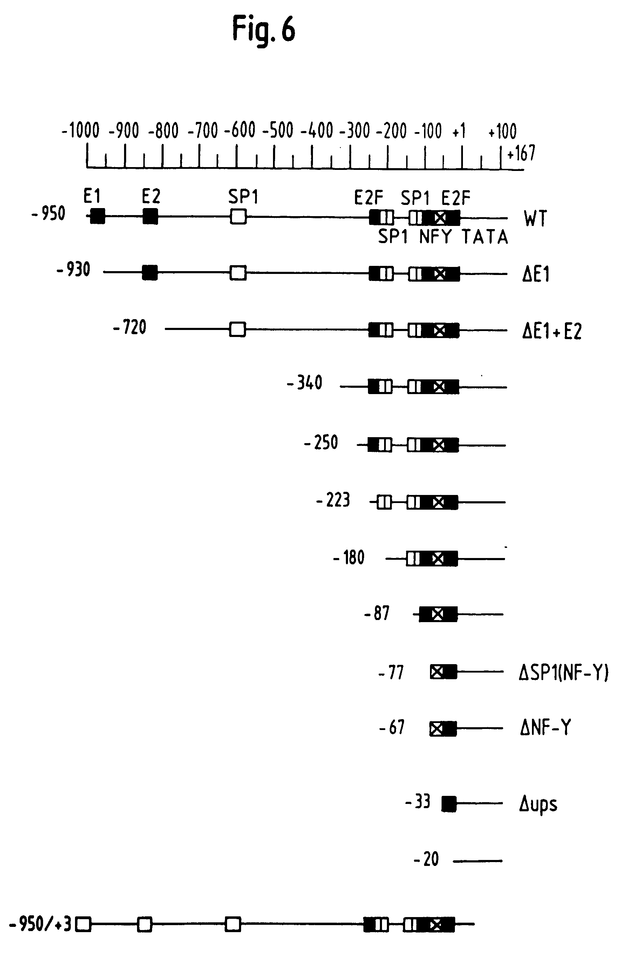

Weitere bevorzugte Teile des erfindungsgemäßen Promotors sind gemäß Tabelle 1 die Nukleotide von ca. -950 bis ca. + 167, von ca. -950 bis ca. + 3, von ca. - 930 bis ca. + 3, von ca. -720 bis ca. + 3, von ca, -340 bis ca. + 3, von ca. - 180 bis ca. + 3, von ca. -100 bis ca. + 3, von ca. -80 bis ca. + 3, von ca. -60 bis ca. + 3 oder von ca. -30 bis ca. + 3, sowie Teile davon, die die entsprechenden funktionellen cis-regulatorischen Elemente gemäß Fig. 6 enthalten, insbesondere 5'- und/oder 3'-Deletionen.

Ein weiterer Gegenstand der vorliegenden Erfindung ist ein Verfahren zum Auffinden von cdc25B-Promotoren, wobei ein erfindungsgemäßer Promotor oder ein Teil davon markiert, vorzugsweise radioktiv markiert wird, und mittels Hybridisierung unter stringenten Bedingungen genomische DNA-Bibliotheken, vorzugsweise aus Säugerzellen, gescreent, d.h. durchsucht werden. Die Herstellung genomischer DNA-Bibliotheken und die Hybridisierung unter stringenten Bedingungen ist dem Fachmann beispielsweise aus Sambrook, J. et. al. (1989) Molecular Cloning. A laboratory manual, Cold Spring Harbour Laboratory New York bekannt. Eine Optimierung der Hybridisierungsbedingungen kann beispielsweise nach Szostak, J.W. et. al. (1979) Hybridization with synthetic oligonucleotides, Methods in Enzymol. 68, 419-482 durchgeführt werden. Zur Isolierung des murinen cdc25B-Promotors kann beispielsweise eine murine, genomische Phagenbank, welche beispielsweise aus dem Mausstamm 129 FVJ, Stratogene, erhalten wurde, mit einer Sonde enthaltend einen Teil der Sequenz gemäß Tabelle 1 (SEQ ID NO:7), vorzugsweise enthaltend die Sequenz SEQ ID NO:4 gescreent werden.

Ein anderer Gegenstand der vorliegenden Erfindung ist ein Nukleinsäurekonstrukt enthaltend mindestens einen erfindungsgemäßen Promotor. Vorzugsweise ist die erfindungsgemäße Promotorsequenz des cdc25B Genes mit einem Strukturgen kombiniert, d.h. im allgemeinen mit einem Gen, das für ein Protein oder für eine RNA in Form eines Wirkstoffes kodiert. Im einfachsten Fall kann diese Kombination ein Nukleinsäurekonstrukt darstellen, welches die Nukleotidsequenz des erfindungsgemäßen Promotors für das cdc25B Gen und ein Strukturgen enthält, wobei der Promotor die Transkription des Strukturgens vorzugsweise zellzyklusabhängig aktiviert. Bevorzugt ist der erfindungsgemäße Promotor stromaufwärts von dem Strukturgen angeordnet.

In einer weiteren bevorzugten Ausführungsform ist die 5'-nicht kodierende Region des cdc25B Genes (Nukleotidsequenz von + 1 bis ca. + 167) zwischen dem erfindungsgemäßen Promotor und dem Strukturgen eingefügt.

In einer anderen bevorzugten Ausführungsform ist der erfindungsgemäße Promotor mit mindestens einer weiteren nichtspezifischen, virusspezifischen, metabolischspezifischen, zellspezifischen, zellzyklusspezifischen und/oder zellproliferationsabhängigen Aktivierungssequenz zur Regulierung der Expression eines Strukturgenes kombiniert. Beispiele sind Promotoren, die in Endothelzellen, Peritonealzellen, Pleuralzellen, Ephitelzellen der Haut, Zellen der Lunge, Zellen des Gastrointestinaltraktes, Zellen der Niere und harnableitenden Wege, Muskelzellen, Bindegewebszellen, blutbildende Zellen, Makrophagen, Lymphozyten, Leukämiezellen, Tumorzellen oder Gliazellen aktiviert werden; Promotorsequenzen von Viren wie HBV, HSV, HPV, EBV, HTLV, CMV oder HIV; Promotor- oder Enhancersequenzen, die durch Hypoxie aktiviert werden; zellzyklusspezifischen Aktivierungssequenzen der Gene kodierend für cdc25C, Cyclin A, cdc2, E2F-1, B-myb und DHFR, und/oder Bindesequenzen für zellproliferationsabhängig auftretende oder aktivierte Transkriptionsfaktoren wie Monomere oder Multimere der Myc E-Box.

Für die Kombination des erfindungsgemäßen Promotors mit mindestens einem weiteren Promotor können unterschiedliche Techniken angewandt werden. Derartige Techniken sind beispielsweise in DE19617851.7, DE19639103.2 und DE19651443.6 beschrieben.

In einer weiteren bevorzugten Ausführungsform dieser Erfindung wird für die Kombination des erfindungsgemäßen Promotors mit mindestens einem weiteren Promotor oder Enhancer ein Nukleinsäurekonstrukt gewählt, das den erfindungsgemäßen Promotor in einer Form enthält, bei welcher mindestens eine Bindestelle für einen Transkriptionsfaktor mutiert ist. Durch diese Mutation wird die Initiation der Transkription des Strukturgens blockiert. Weitere Komponenten des Nukleinsäurekonstruktes sind gegebenenfalls das Strukturgen, mindestens eine weitere unspezifisch, zellspezifisch, virusspezifisch, durch Tetrazyklin und/oder zellzyklusspezifisch aktivierbare Promotor- oder Enhancersequenz, welche die Transkription mindestens eines weiteren Strukturgens aktiviert, das für mindestens einen Transkriptionsfaktor kodiert, welcher derartig mutiert ist, daß er an die mutierte(n) Bindestelle(n) des erfindungsgemäßen Promotors bindet und diesen aktiviert, und/oder das für einen Transkriptionsfaktor kodierende Strukturgen.

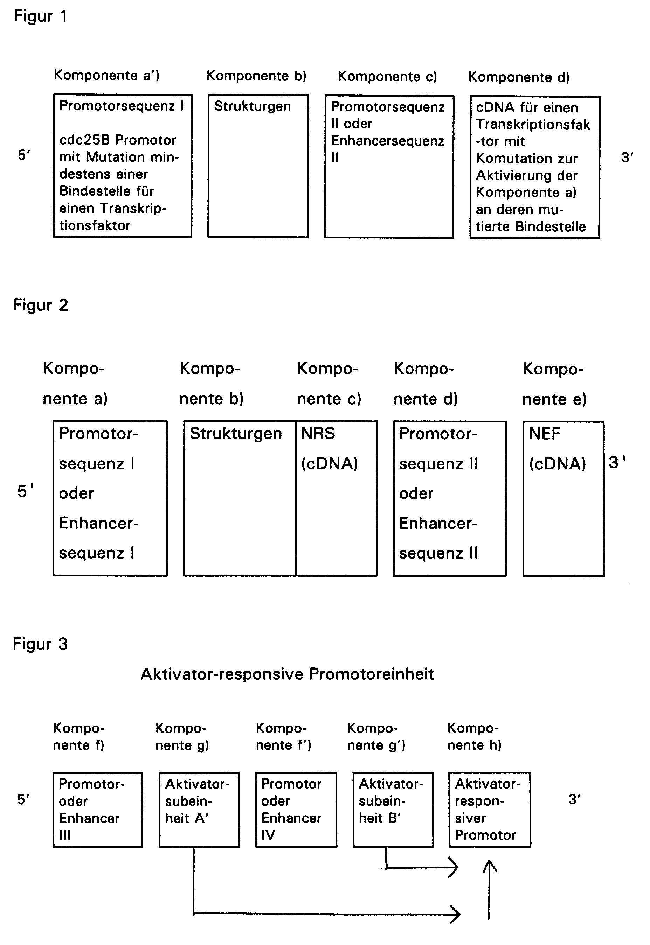

Die Anordnung der einzelnen Komponenten ist beispielhaft durch das Schema in Figur 1 dargestellt.

In einer beispielhaften Ausführungsform dieser Erfindung kann die Mutation eine Mutation der TATA-Box des erfindungsgemäßen Promotors darstellen. Die TATA-Box (TATAAA oder TATATAA) gilt als eine Bindestelle für den Initiationskomplex der im Zellkern vorkommenden RNA-Polymerasen II und III. Die Initiation der Transkription, etwa 30 Basen stromabwärts der TATA-Box, erfolgt durch Bindung des TATA-Box bindenden Proteins (TBP), welches an der Transkriptionsreaktion aller im Zellkern vorhandener RNA-Polymerasen (I, II, III) beteiligt ist. Ein strikt TATA-Box abhängiger Promotor ist beispielsweise der Promotor für das von der RNA-Polymerase III transkribierte U6-Gen, dessen Genprodukt am Spleißvorgang der mRNA beteiligt ist.

Eine Mutation der TATA-Boxsequenz kann beispielsweise TGTATAA sein. Durch diese Mutation wird die DNA-Bindungsstelle des normalen TBP nicht mehr erkannt und das kodierende Gen nicht mehr effizient transkribiert. Bei einer derartigen Mutation stellt das Gen, das für den Transkriptionsfaktor kodiert, eine Nukleinsäuresequenz dar, welche ein komutiertes TBP kodiert. Durch diese Komutation bindet das TBP an die mutierte TATA-Box (z.B. an TGTATAA) und führt damit zur effizienten Transkription des Strukturgens. Derartige Komutationen des TBP-Genes sind beispielsweise von Strubin und Struhl (Cell, 68, 721 (1992)) und von Heard et al. (EMBO J., 12, 3519 (1993)) beschrieben worden.

Eine besonders bevorzugte Ausführungsform ist ein Nukleinsäurekonstrukt enthaltend
(1) den erfindungsgemäßen Promotor des cdc25B-Genes einschließlich TATA-Box, wobei die Sequenz der TATA-Box mutiert ist zu TGTA,
(2) die Sequenz GCCACC,
(3) die cDNA des Signalpeptids des Immunglobulin (Nukleotidsequenz ≤ 63 bis ≥ 107),
(4) die cDNA der β-Glucuronidase (Nukleotidsequenz ≤ 93 bis ≥ 1982),
(5) den Promotor des vWF-Genes (Nukleotidsequenz -487 bis + 247), und
(6) die cDNA des TATA-Box bindenden Proteins (Nukleinsäuresequenz von 1 bis 1001, welche an der Nukleinsäurepositionen 862 (A ausgetauscht gegen T), 889 und 890 (GT ausgetauscht gegen AC) und 895 (C ausgetauscht gegen G) mutiert ist.

In einer weiteren bevorzugten Ausführungsform dieser Erfindung wird für die Kombination des erfindungsgemäßen Promotors mit mindestens einem weiteren Promotor ein Nukleinsäurekonstrukt gewählt, welches multipler Promotor mit nuklearem Retentions-Signal und Export-Faktor benannt wird, und folgende Komponenten enthält:
a) eine erste unspezifische, zellspezifische, virusspezifische, metabolisch und/oder zellzyklusspezifisch aktivierbare Promotor- oder Enhancersequenz (I), die die basale Transkription eines Strukturgens aktiviert,
b) ein Strukturgen,
c) ein nukleares Retentions-Signal (NRS), dessen cDNA am 5'-Ende mit dem 3'-Ende des Strukturgens mittelbar oder unmittelbar verknüpft ist, wobei vorzugsweise das Transkriptionsprodukt des nuklearen Retentions-Signals eine Bindestruktur für einen nuklearen Export-Faktor hat,
d) eine weitere Promotor- oder Enhancersequenz (II), welche die basale Transkription eines nuklearen Export-Faktors (NEF) aktiviert, und
e) eine Nukleinsäure kodierend für einen nuklearen Export-Faktor (NEF), der an das Transkriptionsprodukt des nuklearen Retentions-Signals bindet und hierdurch den Transport des Transkriptionsproduktes des Strukturgens aus dem Zellkern vermittelt.

Im Sinne dieser Erfindung stellt zumindest eine der Promotorkomponenten den erfindungsgemäßen Promotor dar.

Die erste (I) Promotor- oder Enhancersequenz (a) und die zweite (II) Promotor- oder Enhancersequenz (d) können gleich oder unterschiedlich sein und gegebenenfalls unspezifisch, zellspezifisch, virusspezifisch oder metabolisch, insbesondere durch Hypoxie, aktivierbar sein oder einen weiteren zellzyklusspezifischen Promotor darstellen.

Die Anordnung der einzelnen Komponenten ist beispielsweise in Figur 2 wiedergegeben.

Bei den erfindungsgemäßen Nukleinsäurekonstrukten können die Komponenten d) und e) stromaufwärts oder stromabwärts der Komponenten a), b) und c) gelegen sein (siehe auch Fig. 2).

Bevorzugt wird das Gen, welches für das nukleare Retentions-Signal (NRS) kodiert, ausgewählt aus dem Rev-responsive Element (RRE) von HIV-1 oder HIV-2, das RRE-äquivalente Retentions-Signal von Retroviren oder dem RRE-äquivalenten Retentions-Signal des HBV.

Das Gen, welches für den nuklearen Export-Faktor (NEF) kodiert, ist vorzugsweise ein Gen ausgewählt aus dem Rev-Gen der Viren HIV-1, HIV-2, Visna-Maedi Virus, Caprine arthritis encephalitis Virus, dem Virus der infektiösen Anämie des Pferdes, dem Immundefizienzvirus der Katze, Retroviren, HTLV, dem Gen kodierend für das hnRNP-A1-Protein oder dem Gen kodierend für den Transkriptionsfaktor TFIII-A.

In einer weiteren bevorzugten Ausführungsform ist bei den erfindungsgemäßen Nukleinsäurekonstrukten mindestens eine Promotor- oder Enhancersequenz (Komponente a) oder d) ein Genkonstrukt, welches Aktivator-responsive Promotoreinheit benannt wird, und vorzugsweise aus folgenden Komponenten besteht:
f) eine oder mehrere gleiche oder unterschiedliche Promotor- oder Enhancersequenz(en), die beispielsweise zellzyklusspezifisch, zellproliferationsabhängig, metabolisch, zellspezifisch oder virusspezifisch oder sowohl zellzyklusspezifisch als auch metabolisch, zellspezifisch oder virusspezifisch (sogenannte chimäre Promotoren) aktivierbar ist bzw. sind,
g) eine oder mehrere, gleiche oder unterschiedliche Aktivatorsubeinheit(en), welche jeweils stromabwärts von den Promotor- oder Enhancersequenzen gelegen und durch diese in ihrer basalen Transkription aktiviert wird bzw. werden,
h) einen Aktivator-responsiven Promotor, welcher durch die Expressionsprodukte von einer oder mehreren Aktivatorsubeinheit(en) aktiviert wird.

Die Anordnung der einzelnen Komponenten einer bevorzugten Aktivatorresponsiven Promotoreinheit ist in Figur 3 wiedergegeben.

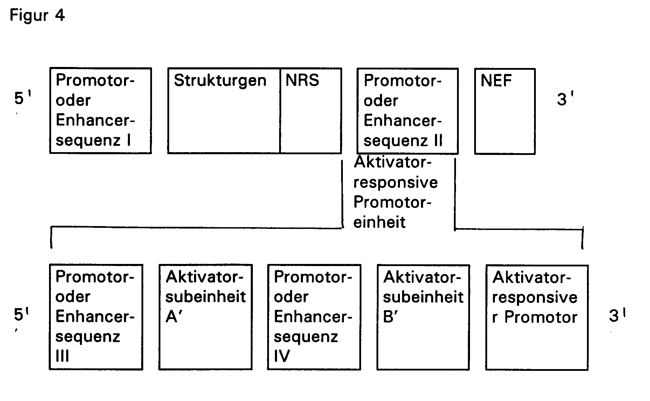

Die Einfügung einer bevorzugten Aktivator-responsiven Promotoreinheit in ein erfindungsgemäßes Nukleinsäurekonstrukt ist beispielsweise in Figur 4 wiedergegeben.

In diesen in den Figuren 3) und 4) beispielhaft dargestellten Aktivatorresponsiven Promotoreinheiten kann mindestens ein Promotor (I, II, III oder IV) den erfindungsgemäßen Promotor darstellen.

In einer bevorzugten Ausführungsform können die Aktivator-responsive Promotoreinheiten Bindesequenzen für chimäre Transkriptionsfaktoren aus DNA-Bindungsdomänen, Protein-Proteininteraktionsdomänen und Transaktivierungsdomänen darstellen. Alle in der vorliegenden Erfindung genannten Transkriptionsfaktorbindungsstellen können einfach (Monomere) oder in mehreren Kopien (Multimere beispielsweise bis zu ca. 10 Kopien) vorliegen.

Ein Beispiel für einen Aktivator-responsiven Promotor (h), der durch zwei Aktivatorsubeinheiten (g, g') aktiviert wird, stellt der LexA-Operator in Verbindung mit dem SV40-Promotor dar.
Dieser enthält beispielsweise folgende Aktivatorsubeinheiten:
(1) Die erste Aktivatorsubeinheit (g) enthält die cDNA kodierend für das LexA-DNA-Bindeprotein mit den Aminosäuren 1-81 oder 1-202, deren 3'-Ende mit dem 5'-Ende der cDNA kodierend für das Gal80-Protein (Aminosäuren 1-435) verknüpft ist, und
(2) die zweite Aktivatorsubeinheit (g') enthält die cDNA kodierend für die Gal80-Bindungsdomäne des Gal4-Proteins kodierend für die Aminosäuren 851-881, deren 3'-Ende mit dem 5'-Ende der cDNA kodierend für das SV40 large T-Antigens mit den Aminosäuren 126-132 verknüpft ist, deren 3'-Ende mit dem 5'-Ende der cDNA kodierend für die Transaktivierungdomäne des VP16 von HSV-1 mit den Aminosäuren 406-488 verknüpft ist.

Ein weiteres Beispiel für einen Aktivator-responsiven Promotor, der durch zwei Aktivatorsubeinheiten (g, g') aktiviert wird, stellt die Bindesequenz für das Gal4-Protein in Verbindung mit dem SV40-Promotor dar.
Dieser enthält beispielsweise folgende Aktivatorsubeinheiten:
(1) Die erste Aktivatorsubeinheit (g) enthält die cDNA kodierend für die DNA-Bindedomäne des Gal4-Proteins (Aminosäuren 1-147), deren 3' Ende mit dem 5' Ende der cDNA für das Gal80-Protein (Aminosäuren 1-435) verknüpft ist, und
(2) die zweite Aktivatorsubeinheit (g') enthält die cDNA kodierend für die Gal80-Bindungsdomäne von Gal4 (Aminosäuren 851 bis 881), deren 3' Ende mit dem 5' Ende der cDNA kodierend für das nukleare Lokalisationssignal von SV40 (SV40 Large T, Aminosäuren 126-132) verknüpft ist, deren 3' Ende mit dem 5' Ende der cDNA kodierend für die Transaktivierungsdomäne des VP16 von HSV-1 mit den Aminosäuren 406-488 verknüpft ist.

Ein weiteres Beispiel für zwei Aktivatorsubeinheiten (g, g'), die den Aktivatorresponsiven Promotor, der die Bindesequenz für das Gal4-Protein und dem SV40-Promotor enthält aktivieren, ist
(1) die Aktivierungseinheit (g) enthaltend die cDNA kodierend für die zytoplasmische Domäne des CD4-T-Zellantigens (Aminosäuren 397-435), deren 5' Ende mit dem 3' Ende der cDNA für die Transaktivierungsdomäne des VP16 von HSV-1 (Aminosäuren 406-488) verknüpft ist, deren 5' Ende wiederum mit dem 3' Ende der cDNA des nuklearen Lokalisationssignals von SV40 (SV40 large T, Aminosäuren 16-132) verknüpft ist, und
(2) die Aktivierungseinheit (g') enthaltend die cDNA kodierend für das nukleare Lokalisationssignals von SV40 (SV40 large T, Aminosäuren 126-132) und die cDNA für die DNA Bindedomäne des Gal4-Proteins (Aminosäuren 1-147), deren 3' Ende mit dem 5' Ende der cDNA für die CD4 Bindesequenz des p56 Ick Proteins (Aminosäuren 1-71) verknüpft ist.

Eine bevorzugte Ausführungsform ist daher ein Nukleinsäurekonstrukt enthaltend
(1) eine oder mehrere, gleiche oder unterschiedliche Aktivatorsubeinheiten, welche von einem Promotor bzw. Enhancer in ihrer basalen Transkription aktiviert wird/werden, und
(2) einen Aktivator-responsiven Promotor, der durch das Expressionsprodukt der genannten Aktivatorsubeinheit aktiviert wird
und eine besonders bevorzugte Ausführungsform ist ein Nukleinsäurekonstrukt enthaltend als eine Aktivatorsubeinheit (A),
(1) den erfindungsgemäßen Promotor,
(2) das nukleare Lokalisationssignal (NLS) von SV40 (SV40 Large T, Aminosäuren 126-132; PKKKRKV),
(3) die saure Transaktivierungsdomäne (TAD) von HSV-1 VP16 (Aminosäuren 406 bis 488), und
(4) die cDNA kodierend für den zytoplasmatischen Teil des CD4 Glycoproteins (Aminosäuren 397-435);
und als eine andere Aktivatorsubeinheit (B),
(1) den Promotor des cdc25C-Gens (Nukleinsäuren -290 bis + 121),
(2) das nukleare Lokalisationssignal (NLS) von SV40 (SV40 Large T; Aminosäuren 126-132 PKKKRKV),
(3) die cDNA für die DNA-Bindedomäne des Gal4 Proteins (Aminosäuren 1 bis 147), und
(4) die cDNA für die CD4 Bindesequenz des p56 Ick Proteins (Aminosäuren 1-71)
sowie den Aktivator-responsive Promotor, mit bis zu ca. 10 Kopien der Bindesequenz für Gal4-Bindeprotein mit der Nukleotidsequenz 5'-CGGACAATGTTGACCG-3' und dem basalen Promotor von SV40 (Nukleotidsequenz 48 bis 5191);
und gegebenenfalls ein Strukturgen, vorzugsweise eine komplette cDNA kodierend für einen Wirkstoff, ein Enzym oder ein Fusionsprotein aus einem Liganden und einem Wirkstoff oder einem Liganden und einem Enzym.

Bei dem Strukturgen handelt es sich in der Regel um ein Gen, das für einen pharmakologisch aktiven Wirkstoff kodiert, der vorzugsweise ausgewählt ist aus Enzyme, Fusionsproteine, Cytokine, Chemokine, Wachstumsfaktoren, Rezeptoren für Cytokine, Rezeptoren für Chemokine, Rezeptoren für Wachstumsfaktoren, antiproliferativ, oder zytostatisch oder apoptotisch wirkende Peptide oder Proteine, Antikörper, oder Antikörperfragmente, Angiogeneseinhibitoren, Peptidhormone, Gerinnungsfaktoren, Gerinnungshemmer, fibrinolytische Peptide oder Proteine, auf den Blutkreislauf wirkende Peptide oder Proteine, Blutplasmaproteine, Antigene von Infektionserregern, wie bakterielle Antigene und parasitäre Antigene, Antigene von Zellen, Antigene von Tumoren, wobei das Antigen eine Immunreaktion bewirkt, Thrombose-induzierende Substanzen, Komplement-aktivierende Proteine, Virushüllproteine und/oder Ribozyme.

Im Falle eines Ribozyms handelt es sich bei dem Strukturgen vorzugsweise um ein Gen, das für ein Ribozym kodiert, welches die mRNA inaktiviert, die für ein Protein ausgewählt aus Zellzykluskontrollproteine, insbesondere Cyclin A, Cyclin B, Cyclin D1, Cyclin E, E2F1-5, cdc2, cdc25C oder DP1, Virusproteine, Cytokine, Wachstumsfaktoren oder deren Rezeptoren kodiert.

In einer anderen bevorzugten Ausführungsform kann es sich bei dem Strukturgen um ein Gen handeln, das für ein Enzym kodiert, welches eine Vorstufe eines Pharmakons in ein Pharmakon spaltet.

In einer weiteren bevorzugten Ausführungsform kann das Strukturgen für ein Ligand-Effektorfusionsprotein kodieren, wobei der Ligand ein Antikörper, ein Antikörperfragment, ein Cytokin, ein Wachstumsfaktor, ein Adhäsionsmolekül oder ein Peptidhormon sein kann und der Effektor ein wie oben beschriebener pharmakologisch aktiver Wirkstoff oder ein Enzym. Beispielsweise kann das Strukturgen ein Ligand-Enzymfusionsprotein kodieren, wobei das Enzym eine Vorstufe eines Pharmakons in ein Pharmakon spaltet und der Ligand an eine Zelloberfläche bindet, bevorzugt an Endothelzellen oder Tumorzellen.

Die Nukleinsäurekonstrukte bestehen vorzugsweise aus DNS. Unter dem Begriff Nukleinsäurekonstrukte werden im allgemeinen künstliche Gebilde aus Nukleinsäuren verstanden, die in den Zielzellen transkribiert werden können. Sie sind bevorzugt in einen Vektor eingefügt, wobei Plasmidvektoren oder virale Vektoren besonders bevorzugt sind.

Im allgemeinen werden diese Vektoren Patienten äußerlich oder innerlich, lokal, peroral, intravesikal, nasal, intrabronchial, intramuskulär, subkutan in eine Körperhöhle, in ein Organ, in den Butkreislauf, in den Atemweg, in den Magen-Darm-Trakt, und/oder in den Urogenitaltrakt verabreicht und dienen der Prophylaxe oder Therapie einer Erkrankung.

Durch die erfindungsgemäßen Nukleinsäurekonstrukte kann ein Strukturgen sowohl zellspezifisch, virusspezifisch, unter bestimmten metabolischen Bedingungen und/oder zellzyklusspezifisch exprimiert werden, wobei es sich bei dem Strukturgen bevorzugt um ein Gen handelt, das für einen pharmakologisch aktiven Wirkstoff oder aber für ein Enzym kodiert, welches eine inaktive Vorstufe eines Pharmakons in ein aktives Pharmakon spaltet. Das Strukturgen kann so gewählt sein, daß der pharmakologisch aktive Wirkstoff oder das Enzym als Fusionsprotein mit einem Liganden exprimiert wird und dieser Ligand an die Oberfläche von Zellen, z.B. proliferierende Endothelzellen oder Tumorzellen, bindet.

Ein weiterer Gegenstand der vorliegenden Erfindung bezieht sich auf ein Verfahren zur Herstellung eines erfindungsgemäßen Nukleinsäurekonstruktes, bei dem die einzelnen Komponenten des Nukleinsäurekonstrinktes miteinander verbunden werden. Die Verbindung der einzelnen Komponenten kann nach allgemein bekannten Verfahren, beispielsweise enzymatisch durch Ligasen, erfolgen.

Ein anderer Gegenstand der vorliegenden Erfindung sind auch Zellen insbesondere von Hefen oder Säugern, die ein erfindungsgemäßes Nukleinsäurekonstrukt enthalten. In einer besonders bevorzugten Ausführungsform werden die Nukleinsäurekonstrukte in Zellinien eingebracht, die dann nach Transfektion zur Expression des Transgens verwendet werden können. Diese Zellen können somit zur Bereitstellung eines Heilmittels für Patienten verwendet werden. Eine bevorzugte Verwendung des erfindungsgemäßen Nukleinsäurekonstruktes besteht in der Behandlung einer Erkrankung, wobei die Bereitstellung des Heilmittels die Einführung eines Nukleinsäurekonstruktes in eine Zielzelle und dessen virus- oder zielzellspezifische oder metabolisch spezifische oder unspezifische und zellzyklusspezifische Expression umfaßt. Die Verabreichung erfolgt im allgemeinen genauso wie bei den erfindungsgemäßen Nukleinsäurekonstrukten und dienen ebenso der Prophylaxe oder Therapie von Erkrankungen.

Zur Herstellung eines Heilmittels können beispielsweise Endothelzellen aus dem Blut gewonnen werden und in vitro mit dem erfindungsgemäßen Nukleinsäurekonstrukt transfiziert werden, um anschließend dem Patienten beispielsweise intravenös wieder injiziert zu werden.

Derartige in vitro transfizierte Zellen können auch in Kombination mit einem erfindungsgemäßen Vektor Patienten verabreicht werden. Diese Kombination hat den Vorteil, daß Zellen und Vektoren jeweils gleichzeitig oder zu unterschiedlichen Zeitpunkten, an gleichen oder an unterschiedlichen Orten verabreicht oder injiziert werden können.

Ein weiterer Gegenstand der vorliegenden Erfindung bezieht sich daher auf die Verwendung eines erfindungsgemäßen Nukleinsäurekonstruktes oder einer erfindungsgemäßen Zelle zur Herstellung eines Heilmittels zur Behandlung einer Erkrankung ausgewählt aus Tumorerkrankungen, Leukämien, Autoimmunerkrankungen, Allergien, Arthritiden, Entzündungen, Organabstoßungen, Transplantat gegen Wirt-Reaktionen, Blutgerinnungserkrankungen, Kreislauferkrankungen, Blutarmut, Infektionen, Hormonerkrankungen und/oder ZNS-Schäden. Insbesondere ist es bevorzugt eine Endothelzelle zur Herstellung eines erfindungsgemäßen Heilmittels zu verwenden.

Die erfindungsgemäßen Nukleinsäurekonstrukte kommen in dieser Form nicht in der Natur vor, d.h. das Strukturgen ist nicht natürlicherweise mit dem erfindungsgemäßen Promotor kombiniert.

Die Promotoren und das Strukturgen werden je nach Verwendungszweck ausgewählt. Die folgenden Beispiele dienen hierbei als Anleitung.

Eine detaillierte Beschreibung der einzelnen Komponenten offenbaren WO96/06940, WO96/06938, WO96/06941, WO96/06939, DE19605274.2, DE19617851.7, DE19639103.2 und DE19651443.6.

### I) Promotorsequenzen

Im Sinne der vorliegenden Erfindung sind als Promotorsequenzen [Komponenten a), c), f) oder f')] Ninkleotidsequenzen zu verwenden, welche im allgemeinen nach Bindung von Transkriptionsfaktoren die Transkription eines am 3'-Ende benachbart gelegenen Strukturgenes aktivieren. Die Wahl der mit dem cdc25B-Promotor zu kombinierende(n) Promotorsequenz(en) richtet sich nach der zu behandelnden Erkrankung und der zu transduzierenden Zielzelle. So kann die zusätzliche Promotorsequenz inneingeschränkt, zielzellspezifisch, unter bestimmten metabolischen Bedingungen, zellzyklusspezifisch oder virusspezifisch aktivierbar sein. Des weiteren können in den Komponenten a), c), f) und/oder f') gleiche oder unterschiedliche Promotorsequenzen eingesetzt werden. Zu den ainszuwählenden Promotorsequenzen gehören beispielsweise: uneingeschränkt aktivierbare Promotoren und Aktivatorsequenzen, wie beispielsweise der Promotor der RNA-Polymerase III, der Promotor der RNA-Polymerase II, der CMV-Promotor und -Enhancer oder der SV40 Promotor; virale Promotor- und Aktivatorsequenzen, wie beispielsweise von HBV, HCV, HSV, HPV, EBV, HTLV, HIV. Bei Verwendung des HIV-Promotors ist vorzugsweise die gesamte LTR-Sequenz einschließlich der TAR-Sequenz [Position -453 bis -80, Rosen et al., Cell 41, 813 (1985)] als virusspezifischer Promotor einzusetzen.

Ferner gehören zu den Promotorsequenzen metabolisch aktivierbare Promotor- und Enhancersequenzen, wie beispielsweise der durch Hypoxie induzierbare Enhancer (Semenza et al., PNAS 88, 5680 (1991); McBurney et al., Nucl. Acids Res. 19, 5755 (1991)); zellzyklusspezifisch aktivierbare Promotoren, wie beispielsweise der Promotor des cdc25C Gens, des Cyclin A Gens, des cdc2 Gens, des B-myb Gens, des DHFR-Gens, des E2F-1 Gens, oder Bindesequenzen für während der Zellproliferation auftretende oder aktivierte Transkriptionsfaktoren, wie beispielsweise Bindesequenzen für c-myc-Proteine, wobei zu diesen Bindesequenzen Monomere oder Multimere der als Myc E-Box bezeichneten Nukleotidsequenz [5'-GGAAGCAGACCACGTGGTCTGCTTCC-3'; Blackwood and Eisenmann, Science 251, 1211, (1991)] zu zählen sind; Tetrazyklin aktivierbare Promotoren, wie beispielsweise der Tetrazyklin-Operator in Kombination mit einem entsprechenden Repressor; chimäre Promotoren, die eine Kombination einer stromaufwärts gelegenen zellspezifisch, metabolisch oder virusspezifisch aktivierbaren Aktivatorsequenz mit einem stromabwärts gelegenen Promotormodul darstellen, welches beispielsweise die Nukleotidsequenz CDE-CHR oder E2FBS-CHR enthält, an welche suppressive Proteine binden, die hierdurch die Aktivierung der stromaufwärts gelegenen Aktivatorsequenz in G₀- und G₁ -Phase des Zellzyklus hemmen können (WO96/06943; Lucibello et al., EMBO J. 14, 12 (1994)); zellspezifisch aktivierbare Promotoren, wie Promotoren oder Aktivatorsequenzen aus Promotoren oder Enhancern von solchen Genen, die für Proteine kodieren, die bevorzugt in den ausgewählten Zellen gebildet werden.

Beispiele von zellspezifisch aktivierbaren Promotoren sind Promotor- und Aktivatorsequenzen, die in Endothelzellen aktiviert werden, wie z.B. die Promotor- und Aktivatorsequenzen der Gene kodierend für den Hirnspezifischen, endothelialen Glucose-1-Transporter, Endoglin, VEGF-Rezeptor-1 (flt-1), VEGF-Rezeptor-2 (flk-1, KDR), til-1 oder til-2, B61-Rezeptor (Eck-Rezeptor), B61, Endothelin, im speziellen Endothelin B oder Endothelin-1, Endothelin-Rezeptoren, insbesondere der Endothelin B-Rezeptor, Mannose-6-Phosphat-Rezeptoren, von Willebrand Faktor, IL-1α, IL-1β, IL-1-Rezeptor, Vascular Cell Adhesion Molecule (VCAM-1) oder synthetische Aktivatorsequenzen, die aus oligomerisierten Bindestellen für Transkriptionsfaktoren bestehen, die preferentiell oder selektiv beispielsweise in Endothelzellen aktiv sind. Ein Beispiel hierfür ist der Transkriptionsfaktor GATA-2, dessen Bindestelle im Endothelin-1 Gen 5'-TTATGT-3' ist [Lee et al., Biol. Chem. 266, 16188 (1991], Dormann et al., J. Biol. Chem. 267, 1279 (1992) und Wilson et al., Mol. Cell Biol. 10, 4854 (1990)]. Weitere zellspezifisch aktivierbare Promotoren sind Promotoren oder Aktivatorsequenzen, die in Zellen in Nachbarschaft aktivierter Endothelzellen aktiviert werden, wie z.B. die Promotor- und Aktivatorsequenzen der Gene kodierend für VEGF, wobei die genregulatorischen Sequenzen für das VEGF-Gen die 5' flankierende Region, die 3' flankierende Region, das c-Src-Gen oder das v-Src-Gen sind, oder Steroid-Hormonrezeptoren und deren Promotorelemente (Truss und Beato, Endocr. Rev. 14, 459 (1993)), insbesondere der Maus-Mammatumor-Virus-Promotor.

Promotoren oder Aktivatorsequenzen, die in Muskelzellen aktiviert werden, insbesondere in glatten Muskelzellen, sind beispielsweise Promotor- und Aktivatorsequenzen der Gene kodierend für Tropomyosin, α-Actin, α-Myosin, Rezeptor für PDGF, Rezeptor für FGF, MRF-4, Phosphofructokinase A, Phosphoglyceratemutase, Troponin C, Myogenin, Rezeptoren für Endothelin A, Desmin, VEGF, wobei die genregulatorischen Sequenzen für das VEGF-Gen bereits oben beschrieben sind oder "artifizielle" Promotoren. Derartige artifizielle Promotoren sind beispielsweise multiple Kopien der (DNA) Bindestelle für muskelspezifische Helix-Loop-Helix (HLH)-Proteine wie der E-Box (Myo D) (z.B. 4x AGCAGGTGTTGGGAGGC, SEQ ID NO.: 1) oder multiple Kopien der DNA Bindestelle für das Zinkfingerprotein GATA-4 des α-Myosin-Heavy Chain Gens (z.B. 5'-GGCCGATGGGCAGATAGAGGGGGCCGATGGGCAGATAGAGG3', SEQ ID NO.: 2). HLH-Proteine sind beispielsweise MyoD, Myf-5, Myogenen, oder MRF4. Die HLH-Proteine sowie GATA-4 zeigen muskelspezifische Transkription nicht nur mit Promotoren von muskelspezifischen Genen, sondern auch im heterologen Kontext, so auch mit artifiziellen Promotoren.

Promotoren und Aktivatorsequenzen, die in Gazellen aktiviert werden, sind im besonderen die genregulatorischen Sequenzen bzw. Elemente aus Genen, die beispielsweise für folgende Proteine kodieren: das Schwannzell-spezifische Protein Periaxin, Glutaminsynthetase, das Gliazell-spezifische Protein (Glial fibrillary acid protein = GFAP), das Gliazellprotein S100b, IL-6, CNTF, 5-HT-Rezeptoren, TNFα, IL-10, Insulin-like Growth Factor Receptor I and II oder VEGF, wobei die die genregulatorischen Sequenzen für das VEGF-Gen bereits oben aufgeführt worden sind.

Promotoren und Aktivatorsequenzen, die in blutbildenden Zellen aktiviert werden, sind Promotorsequenzen für Gene eines Cytokins oder seines Rezeptors, die in blutbildenden Zellen oder in benachbarten Zellen, wie beispielsweise dem Stroma, exprimiert sind.

Hierzu gehören Promotorsequenzen für beispielsweise folgende Cytokine und ihre Rezeptoren: Stem Cell Factor-Receptor, Stem Cell Factor, IL-1α IL-1-Rezeptor, IL-3, IL-3-Rezeptor (α-subunit), IL-3-Rezeptor (β-subunit), IL-6, IL-6-Rezeptor, GM-CSF, GM-CSF-Rezeptor (α-Kette), Interferon Regulatory Factor 1 (IRF-1), wobei der Promotor von IRF-1 durch IL-6 gleichermaßen aktiviert wird wie durch IFNγ oder IFNβ, Erythropoietin oder Erythropoietin-Rezeptor.

Promotoren und Aktivatorsequenzen, die in Lymphozyten und/oder Makrophagen aktiviert werden, sind beispielsweise die Promotor- und Aktivatorsequenzen der Gene für Cytokine, Cytokinrezeptoren und Adhäsionsmoleküle und Rezeptoren für das Fc-Fragment von Antikörpern.

Hierzu gehören beispielsweise Promotorsequenzen für folgende Proteine: IL-1-Rezeptor, IL-1α, IL-1β, IL-2, IL-2-Rezeptor, IL-3, IL-3-Rezeptor (α-subunit), IL-3-Rezeptor (β-subunit), IL-4, IL-4-Rezeptor, IL-5, IL-6, IL-6-Rezeptor, Interferon Regulatory Factor 1 (IRF-1), wobei der Promotor von IRF-1 durch IL-6 gleichermaßen aktiviert wird wie durch IFNγ oder IFNβ, IFNγ Responsive Promotor, IL-7, IL-8, IL-10, IL-11, IFNγ, GM-CSF, GM-CSF-Rezeptor (α-Kette), IL-13, LIF, Makrophagen-Colony Stimulating Factor (M-CSF)-Rezeptor , Typ I und II Makrophagen Scavenger Rezeptoren, MAC-1 (Leukozytenfunktionsantigen), LFA-1α (Leukozytenfunktionsantigen) oder p150,95 (Leukozytenfunktionsantigen).

Promotor- und Aktivatorsequenzen, die in Synovialzellen aktiviert werden, sind beispielsweise die Promotorsequenzen für Matrix-Metalloproteinasen (MMP), beispielsweise für MMP-1 (interstitielle Kollagenase) oder MMP-3 (Stromelysin/Transin).

Hierzu gehören des weiteren die Promotorsequenzen für Tissue Inhibitors of Metalloproteinases (TIMP), beispielsweise TIMP-1, TIMP-2 oder TIMP-3.

Promotoren und Aktivatorsequenzen, die in Leukämiezellen aktiviert werden, sind beispielsweise Promotoren für c-myc, HSP-70, bcl-1/cyclin D-1, bcl-2, IL-6, IL-10, TNFα, TNFβ, HOX-11, BCR-Abl, E2A-PBX-1, PML-RARA (Promyelocytic Leukemia - Retinoic Acid Receptor) oder c-myc, wobei c-myc-Proteine an Multimere der als Myc E-Box bezeichneten Nukleotidsequenz (5'-GGAAGCAGACCAGCTGGTCTGCTTCC-3', SEQ ID NO.: 3) binden und diese aktivieren.

Promotoren oder Aktivatorsequenzen, die in Tumorzellen aktiviert werden, ist beispielsweise eine genregulatorische Nukleotidsequenz, mit der Transkriptionsfaktoren, gebildet oder aktiv in Tumorzellen, interagieren.

Im Sinne dieser Erfindung zählen zu den bevorzugten Promotoren oder Aktivatorsequenzen genregulatorische Sequenzen bzw. Elemente aus Genen, die besonders in Krebszellen oder Sarkomzellen gebildete Proteine kodieren. So wird bei kleinzelligen Bronchialkarzinomen bevorzugt der Promotor des N-CAM-Proteins, bei Ovarialkarzinomen der Promotor des "Hepatitis growth factor"-Rezeptors oder des L-Plastin und bei Pankreaskarzinomen der Promotor des L-Plastins oder des polymorphen epithelialen Mucins (PEM) verwendet.

### II) Nukleare Exportsignale und nukleare Exportfaktoren

Bei einer bevorzugten Ausführungsform ist das nukleare Retentions-Signal (NRS) eine Nukleotidsequenz, die den Transport einer mit ihr verknüpften premessenger RNA durch die Kernmembran behindert, die jedoch andererseits auch eine Bindestruktur für ein Exportprotein darstellt. Dieses Exportprotein vermittelt den Transport der ein NRS enthaltende premessenger oder messenger RNA aus dem Zellkern in das Zytoplasma. Eine, das NRS enthaltende premessenger oder messenger RNA wird somit durch Bindung an das Exportprotein aus dem Zellkern ausgeschleust (Fischer et al., Cell, 82, 475 (1995)).

Bei den nuklearen Exportsignalen (NES) handelt es sich vorzugsweise um die Rev-Responsive Element (RRE)-Sequenz von Retroviren. Bei HIV-1 ist diese RRE eine 243 Nukleotide (Nukleotide 7362-7595) umfassende Sequenz im env-Gen. Das nukleare Exportsignal (NES) kann jedoch auch jede homologe und/oder funktionell ähnliche (analoge) Nukleotidsequenz sein, so beispielsweise das RRE-equivalente Element des HBV-Virus (Huang et al., Mol. Cell Biol., 13, 7476 (1993)).

Bei den erfindungsgemäßen Nukleinsäurekonstrukten ist der nukleare Exportfaktor (NEF) eine Nukleotidsequenz, die für ein Protein kodiert, welches an die mRNA des NRS bindet und den Transport der ein NRS enthaltende premessenger RNA oder messenger RNA aus dem Zellkern in das Zytoplasma (oder aus dem Zytoplasma in den Zellkern) vermittelt. Im besonderen wird das rev-Gen von Retroviren, speziell vom HIV-1 oder HIV-2 Virus verwendet. Das rev-Protein des rev-Genes von Retroviren bindet mit seiner N-terminalen Domäne an das RRE in der pre-mRNA. Durch die Bindung zwischen dem RRE und dem rev-Protein wird der Transport von "nonspliced" premessenger RNA, aber auch jeder weiteren RNA, die ein RRE enthält, vom Zellkern in das Zytoplasma ermöglicht und damit die Translation erheblich verstärkt.

Im Sinne der vorliegenden Erfindung können als NEF auch Ninkleotidsequenzen verwendet werden, die Proteine kodieren, die homolog und funktionell ähnlich dem rev-Protein von HIV-1 sind (Bogerd et al., Cell, 82, 485 (1995)), wie beispielsweise das rev-Gen des Visna-Maedi Virus (VMV) oder das rev-Gen des Caprine arthritis encephalitis Virus (CAEV). Es können jedoch auch solche Gene eingesetzt werden, die Proteine kodieren, die zwar nur eine geringe oder keine Homologie zum rev-Protein besitzen, jedoch funktionell ähnlich dem rev-Protein von HIV-1 sind. Hierzu zählen z.B. das rev-Gen des HTLV-1, das rev-Gen des Virus der infektiösen Anämie des Pferdes (EIAV) und des Immundefizienzvirus der Katze (FIV).

In einer alternativen Ausführungsform kann es sich bei den NEF auch um Nukleotidsequenzen für Proteine handeln, welche eine Ausschleusung von RNA aus dem Kern bewirken, ohne daß diese durch ein NRS im Kern zurückgehalten wird. Hierzu zählen beispielsweise der Transkriptionsfaktor TFIIIA oder das heterogene nukleare Ribonukleoprotein A1 (hnRNPA1-Protein). Des weiteren gehören zu den nuklearen Transportproteinen im erweiterten Sinne das "heat shock protein 70" (hsc70) oder der Proteinkinaseinhibitor CPKI.

Gemeinsam ist dem NEF und seinen homologen and analogen Proteinen eine mehr aminoterminal gelegene Domäne für die Bindung des monomeren Proteins an die RNA des NRS und eine meist Leucin-reiche Domäne (Ausnahme hiervon ist hnRNPA1), welche für die Transportfunktion des NEF notwendig ist.

Im Sinne dieser Erfindung steht die Expression des NEF-Genes unter der Kontrolle einer stromaufwärts am 5'-Ende des NEF-Genes gelegenen Promotorsequenz, wie oben bereits näher beschrieben.

### III) Strukturgene

Im Sinne der Erfindung kodieren die Strukturgene [Komponente b)] für einen Wirkstoff zur Prophylaxe und/oder Therapie einer Erkrankung. Strukturgene und Promotorsequenzen sind im Hinblick auf die Art der Therapie der Erkrankung und unter Berücksichtigung der zu transduzierenden Zielzelle auszuwählen.

Beispielsweise sind bei folgenden Erkrankungen folgende Kombinationen von Promotorsequenzen und Strukturgenen zu wählen. Eine detaillierte Beschreibung findet man bereits in den Patentanmeldungen WO96/06940, DE19605274.2, DE19617851.7, DE19639103.2 und DE19651443.6, auf die Bezug genommen wird.

Für die Therapie von Tumoren werden beispielsweise Zielzellen: proliferierende Endothelzellen, der Endothelzelle benachbarte Stromazellen und Muskelzellen oder Tumorzellen oder Leukämiezellen ausgewählt. Die Promotoren sind endothelzellspezifisch und zellzyklusspezifisch oder zellunspezifisch oder muskelzellspezifisch und zellzyklusspezifisch oder tumorzellspezifisch (solide Tumoren, Leukämien) und zellzyklusspezifisch.

Bei der Auswahl der Strukturgene für Inhibitoren der Zellproliferation, zum Beispiel für das Retinoblastomprotein (pRb = p110) oder die verwandten p107 und p130 Proteine, kann folgende Strategie gewählt werden:

Das Retinoblastomprotein (pRb/p110) und die verwandten p107 und p130 Proteine werden durch Phosphorylierung inaktiviert. Bevorzugt sind solche Gene dieser Zellzyklusinhibitoren zu verwenden, welche Mutationen für die Inaktivierungsstellen der exprimierten Proteine aufweisen, ohne daß diese hierdurch in ihrer Funktion beeinträchtigt werden. Beispiele für diese Mutationen wurden für das p110 beschrieben. In analoger Weise kann die DNA-Sequenz für das p107 Protein oder das p130 Protein mutiert werden. Ein weiterer Inhibitor der Zellproliferation ist das p53 Protein. Das Protein p53 wird in der Zelle entweder durch Bindung an spezielle Proteine, wie beispielsweise MDM2, oder durch Oligomerisierung des p53 über das dephosphorylierte C-terminale Serin inaktiviert. Bevorzugt wird somit eine DNA-Sequenz für ein p53 Protein verwendet, welches C-terminal um das Serin 392 verkürzt ist. Weitere Inhibitoren sind das p21 (WAF-1), das p16 Protein, andere cdk-Inhibitoren, das GADD45 Protein oder das bak Protein.

Strukturgene für Gerinnung induzierende Faktoren und Angiogeneseinhibitoren kodieren zum Beispiel für Plasminogenaktivatorinhibitor-1 (PAI-1), PAI-2, PAI-3, Angiostatin, Interferone (IFNα, IFNβ oder IFNγ), Platelet factor 4, IL-12, TIMP-1, TIMP-2, TIMP-3, Leukemia Inhibitory Factor (LIF) oder Tissue Factor (TF) und dessen gerinnungsaktive Fragmente.

Strukturgene für zytostatische und zytotoxische Proteine kodieren zum Beispiel für Perforin, Granzym, IL-2, IL-4, IL-12, Interferone, wie beispielsweise IFN-α, IFNβ oder IFNγ, TNF, wie TNFα oder TNFβ, Oncostatin M, Sphingomyelinase oder Magainin und Magainin-Derivate.

Strukturgene, die für zytostatische oder zytotoxische Antikörper und für Fusionsproteine zwischen antigenbindenden Antikörperfragmenten mit zytostatischen, zytotoxischen oder entzündungserregenden Proteinen oder Enzymen kodieren, können nach folgender Strategie ausgewählt werden:

Zu den zytostatischen oder zytotoxischen Antikörpern gehören z.B. solche, die gegen Membranstrukturen von Endothelzellen wie sie beispielsweise von Burrows et al. (Pharmac. Ther., 64, 155 (1994)), Hughes et al., (Cancer Res., 49, 6214 (1989)) und Maruyama et al., (PNAS USA, 87, 5744 (1990)) beschrieben wurden, gerichtet sind. Insbesondere zählen hierzu Antikörper gegen die VEGF-Rezeptoren. Des weiteren gehören hierzu zytostatische oder zytotoxische Antikörper, die gegen Membranstrukturen auf Tumorzellen gerichtet sind. Derartige Antikörper wurden zum Beispiel von Sedlacek et al., Contrib. to Oncol., 32, Karger Verlag, München (1988) und Contrib. to Oncol., 43, Karger Verlag, München (1992) übersichtlich dargestellt. Weitere Beispiele stellen Antikörper gegen Sialyl Lewis; gegen Peptide auf Tumoren, welche von T-Zellen erkannt werden; gegen von Onkogenen exprimierte Proteine; gegen Ganglioside wie GD3, GD2, GM2, 9-0-acetyl GD3, Fucosyl GM1; gegen Blutgruppenantigene und deren Vorläufer; gegen Antigene auf dem polymorphen epithelialen Mucin; gegen Antigene auf Heat Shock Proteinen, dar. Des weiteren gehören hierzu Antikörper, die gegen Membranstrukturen von Leukämiezellen gerichtet sind. Eine große Anzahl derartiger monoklonaler Antikörper sind bereits für diagnostische und therapeutische Verfahren beschrieben worden (Übersichten bei Kristensen, Danish Medical Binlletin, 47, 52 (1994); Schranz, Therapia Hungarica, 38, 3 (1990); Drexler et al., Leuk. Res., 10, 279 (1986); Naeim, Dis., Markers, 71 (1989); Stickney et al., Curr.. Opin. Oncol., 4, 847 (1992); Drexler et al., Blut, 57, 327 (1988); Freedman et al., Cancer Invest., 9, 69 (1991)). Je nach Typ der Leukämie sind als Liganden beispielsweise monoklonale Antikörper oder deren antigenbindende Antikörperfragmente, die gegen folgende Membranantigene gerichtet sind geeignet:

| Zellen | Membranantigen |
|---|---|
| AML | CD13 |
| | CD15 |
| | CD33 |
| | CAMAL |
| | Sialosyl-Le |
| B-CLL | CD5 |
| | CD1c |
| | CD23 |
| | Idiotypen und Isotypen der Membranimmunglobuline |
| T-CLL | CD33 |
| | M38 |
| | IL-2-Rezeptoren |
| | T-Zell-Rezeptoren |
| ALL | CALLA |
| | CD19 |
| | Non-Hodgkin Lymphoma |

Die Humanisierung muriner Antikörper, die Herstellung und Optimierung der Gene für Fab und rekombinante Fv Fragmente erfolgt entsprechend der dem Fachmann bekannten Technik (Winter et al., Nature, 349, 293 (1991); Hoogenbooms et al., Rev. Tr. Transfus. Hemobiol., 36, 19 (1993); Girol. Mol. Immunol., 28, 1379 (1991) oder Huston et al., Intern. Rev. Immunol., 10, 195 (1993)). Die Fusion der rekombinanten Fv-Fragmente mit Genen für zytostatische, zytotoxische oder entzündungserregende Proteinen oder Enzymen erfolgt gleichermaßen entsprechend dem dem Fachmann bekannten Stand der Technik.

Strukturgene, die für Fusionsproteine von Zielzell-bindenden Liganden mit zytostatischen und zytotoxischen Proteinen kodieren können nach folgender Strategie ausgewählt werden. Zu den Liganden gehören z.B. alle Substanzen, welche an Membranstrukturen oder Membranrezeptoren auf Endothelzellen binden. Beispielsweise gehören hierzu Cytokine, wie beispielsweise IL-1 oder Wachstumsfaktoren oder deren Fragmente bzw. Teilsequenzen von ihnen, die an Rezeptoren exprimiert durch Endothelzellen binden, wie beispielsweise PDGF, bFGF, VEGF, TGF.

Des weiteren gehören hierzu Adhäsionsmoleküle, welche an aktivierte und/oder proliferierende Endothelzellen binden. Hierzu gehören beispielsweise SLex, LFA-1, MAC-1, LECAM-1, VLA-4 oder Vitronectin. Hierzu gehören des weiteren Substanzen, welche an Membranstrukturen oder Membranrezeptoren von Tumor- oder Leinkämiezellen binden. Beispielsweise gehören hierzu Wachstumsfaktoren oder deren Fragmente bzw. Teilsequenzen von ihnen, die an Rezeptoren exprimiert durch Leukämiezellen oder Tumorzellen binden. Derartige Wachstumsfaktoren wurden bereits beschrieben (Übersichten bei Cross et al., Cell, 64, 271 (1991), Aulitzky et al., Drugs, 48, 667 (1994), Moore, Clin. Cancer Res., 1, 3 (1995), Van Kooten et al., Leuk. Lymph., 27 (1993)). Die Fusion der Gene dieser an die Zielzelle bindenden Liganden mit Genen von zytostatischen, zytotoxischen oder entzündungserregenden Proteinen oder Enzymen erfolgt entsprechend dem Stand der Technik mit den dem Fachmann bekannten Methoden.

Strukturgene für Induktoren von Entzündungen kodieren, zum Beispiel für IL-1, IL-2, RANTES (MCP-2), monocyte chemotactic and activating factor (MCAF), IL-8, macrophage inflammatory protein-1 (MIP-1α, -β), neutrophil activating protein-2 (NAP-2), IL-3, IL-5, human leukemia inhibitory factor (LIF), IL-7, IL-11, IL-13, GM-CSF, G-CSF, M-CSF, Cobra venom factor (CVF) oder Teilsequenzen vom CVF, welchem dem menschlichen Komplementfaktor C3b funktionell entsprechen, d.h. welche an den Komplementfaktor B binden können und nach Spaltung durch den Faktor D eine C3 Konvertase darstellen, der menschliche Komplementfaktor C3 oder seine Teilsequenz C3b, Spaltprodukte des menschlichen Komplementfaktors C3, welche funktionell und strukturell dem CVF ähneln, oder bakterielle Proteine, welche Komplement aktivieren oder Entzündungen auslösen, wie beispielsweise Porine von Salmonella typhi murium, "clumping" Faktoren von Staphylococcus aureus, Moduline besonders von gram-negativen Bakterien, "Major outer membrane protein" von Legionellen oder von Haemophilus influenza Typ B oder von Klebsiellen oder M-Moleküle von Streptokokken Gruppe G.

Strukturgene, die für Enzyme für die Aktivierung von Vorstufen von Zytostatika kodieren, zum Beispiel für Enzyme, welche inaktive Vorsubstanzen (Prodrugs) in aktive Zytostatika (Drugs) spalten, und die jeweils zugehörigen Prodrugs und Drugs sind bereits von Deonarain et al. (Br. J. Cancer, 70, 786 (1994)), Mullen, Pharmac. Ther., 63, 199 (1994)) und Harris et al. (Gene Ther., 1, 170 (1994)) übersichtlich beschrieben worden. Beispielsweise ist die DNA-Sequenz eines der folgenden Enzyme zu verwenden: Herpes Simplex Virus Thymidinkinase, Varizella Zoster Virus Thymidinkinase, bakterielle Nitroreduktase, bakterielle β-Glucuronidase, pflanzliche β-Glucuronidase aus Secale cereale, humane β-Glucuronidase, humane Carboxypeptidase (CB) zum Beispiel CB-A der Mastzelle, CB-B, des Pankreas oder bakterielle Carboxypeptidase, bakterielle β-Laktamase, bakterielle Cytosinedeaminase, humane Katalase bzw. Peroxidase, Phosphatase, im besonderen humane alkalische Phosphatase, humane saure Prostataphosphatase oder Typ 5 saure Phosphatase, Oxidase, im besonderen humane Lysyloxidase oder humane saure D-Aminooxidase, Peroxidase, im besonderen humane Glutathion-Peroxidase, humane eosinophile Peroxidase oder humane Schilddrüsen-Peroxidase, oder Galaktosidase.

Die Therapie von Autoimmunerkrankungen und Entzündungen ist zudem WO/06941 und DE19651443.6, auf die Bezug genommen wird, beschrieben.

Als Zielzellen eignen sich z.B. proliferierende Endothelzellen, Makrophagen und/oder Lymphozyten oder Synovialzellen. Die Promotoren sind z.B. endothelzellspezifisch und zellzyklusspezifisch oder makrophagen- und/oder lymphozytenspezifisch und/oder zellzyklusspezifisch oder synovialzellspezifisch und/oder zellzyklinsspezifisch.

Die Strukturgene für die Therapie von Allergien kodieren zum Beispiel für IFNβ, IFNγ, IL-10, Antikörper bzw. Antikörperfragmente gegen IL-4, lösliche IL-4-Rezeptoren, IL-12 oder TGFβ.

Die Strukturgene für die Verhinderung der Abstoßung von transplantierten Organen kodieren zum Beispiel für IL-10, TGFβ, lösliche IL-1-Rezeptoren, lösliche IL-2-Rezeptoren, IL-1-Rezeptorantagonisten, lösliche IL-6-Rezeptoren oder immunsuppressive Antikörper oder deren V_{H} und V_{L} enthaltende Fragmente oder deren über einen Linker verbundene V_{H}- und V_{L}-Fragmente. Immunsuppressive Antikörper sind beispielsweise Antikörper, die für den T-Zell-Rezeptor oder seinen CD3-Komplex, CD4 oder CD8, des weiteren den IL-2-Rezeptor, IL-1-Rezeptor oder IL-4-Rezeptor oder die Adhäsionsmoleküle CD2, LFA-1, CD28 oder CD40 spezifisch sind.

Die Strukturgene für die Therapie von Antikörper-mediierten Autoimmunerkrankungen kodieren zum Beispiel für TGFβ, IFNα, IFNβ, IFNγ, IL-12, lösliche IL-4-Rezeptoren, lösliche IL-6-Rezeptoren oder immunsuppressive Antikörper oder dessen V_{H} und V_{L}-enthaltende Fragmente.

Die Struktinrgene für die Therapie von Zell-mediierten Autoimmunerkrankungen kodieren zum Beispiel für IL-6, IL-9, IL-10, IL-13, TNFα oder TNFβ, IL-13 oder einen immunsuppressiven Antikörper oder dessen V_{H}- und V_{L}-enthaltende Fragmente.

Die Strukturgene für Inhibitoren der Zellproliferation, zytostatische oder zytotoxische Proteine und Enzyme für die Aktivierung von Vorstufen von Zytostatika wurden bereits oben zu der Therapie von Tumoren aufgeführt.

In gleicher Form wie dort bereits beschrieben, können im Sinne der vorliegenden Erfindung Strukturgene verwendet werden, welche für Fusionsproteine aus Antikörpern bzw. Fab oder rekombinanten Fv-Fragmenten dieser Antikörper oder anderen Liganden spezifisch für die Zielzelle und den o.a. Cytokinen, Wachstumsfaktoren, Rezeptoren, zytostatischen oder zytotoxischen Proteinen und Enzymen kodieren.

Für die Therapie der Arthritis werden im Sinne der Erfindung Strukturgene ausgewählt, deren exprimiertes Protein die Entzündung beispielsweise im Gelenk direkt oder indirekt hemmt und/oder die Rekonstitution von extrazellulärer Matrix (Knorpel, Bindegewebe) im Gelenk fördert.

Hierzu gehören zum Beispiel IL-1-Rezeptorantagonist (IL-1-RA), da IL-1-RA die Bindung von IL-1α, β inhibiert, löslicher IL-1-Rezeptor, da löslicher IL-1-Rezeptor IL-1 bindet und inaktiviert, IL-6, da IL-6 die Sekretion von TIMP und Superoxiden erhöht und die Sekretion von IL-1 und TNFα durch Synovialzellen und Chondrozyten vermindert, löslicher TNF-Rezeptor, da löslicher TNF-Rezeptor TNF bindet und inaktiviert, IL-4, da IL-4 die Bildung und Sekretion von IL-1, TNFα und MMP inhibiert, IL-10, da IL-10 die Bildung und Sekretion von IL-1, TNFα und MMP inhibiert und die Sekretion von TIMP erhöht, Insulin-like growth factor (IGF-1), da IGF-1 die Synthese von extrazellulärer Matrix stimuliert, TGFβ, im speziellen TGFβ1 und TGFβ2, da TGFβ die Synthese von extrazellulärer Matrix stimuliert, Superoxiddismutase oder TIMP, im speziellen TIMP-1, TIMP-2 oder TIMP-3.

Die Therapie der mangelhaften Bildung von Zellen des Blutes wurde bereits in WO96/06941, auf die Bezug genommen wird, näher beschrieben. Als Zielzellen eignen sich z.B. proliferierende, unreife Zellen des blutbildenden Systems oder Stromazellen benachbart den blutbildenden Zellen. Die Promotoren sind z.B. spezifisch für blutbildende Zellen und/oder Zellzyklusspezifisch oder zellunspezifisch und zellzyklusspezifisch.

Ein Strukturgen für die Therapie der Anämie kodiert zum Beispiel für Erythropoietin. Strukturgene für die Therapie der Leukopenie kodieren zum Beispiel für G-CSF, GM-CSF oder M-CSF. Strukturgene für die Therapie der Thrombozytopenie kodieren zum Beispiel für IL-3, Leukemia Inhibitory Factor (LIF), IL-11 oder Thrombopoietin.

Zur Therapie von Schäden des Nervensystems sind als Zielzellen: Gliazellen oder proliferierende Endothelzellen geeignet. Die Promotoren sind in diesem Fall Gliazell-spezifisch und zellzyklusspezifisch oder Endothelzell-spezifisch und Zellzyklusspezifisch oder unspezifisch und Zellzyklusspezifisch.

Die Strukturgene für neuronale Wachstumsfaktoren kodieren zum Beispiel für FGF, Nerve growth factor (NGF), Brain-derived neurotrophic factor (BDNF), Neurotrophin-3 (NT-3), Neurotrophin-4 (NT-4), Ciliary neurotrophic factor (CNTF) oder Glial cell derived growth factor (GDNF). Die Strukturgene für Enzyme kodieren zum Beispiel für Tyrosinhydroxylase oder Dopadecarboxylase. Die Strukturgene für Cytokine und deren Inhibitoren, welche die neurotoxische Wirkung von TNFα inhibieren oder neutralisieren kodieren zum Beispiel für TGFβ , lösliche TNF-Rezeptoren, TNF-Rezeptoren neutralisieren TNFα, IL-10, da IL-10 die Bildung von IFNγ, TNFα, IL-2 und IL-4 inhibiert, lösliche IL-1-Rezeptoren, IL-1 -Rezeptor I, IL-1-Rezeptor II, da lösliche IL-1-Rezeptoren die Aktivität von IL-1 neutralisieren, IL-1-Rezeptor-Antagonist oder lösliche IL-6-Rezeptoren.

Die Therapie von Störungen des Blutgerinnungs- und Blutkreislaufsystems wurde bereits in den Patentanmeldungen WO96/06938, DE19617851.7 und DE19639103.2, auf welche Bezug genommen wird, näher beschrieben. Als Zielzellen eignen sich z.B. Endothelzellen, proliferierende Endothelzellen, somatische Zellen in Nachbarschaft von Endothelzellen und glatte Muskelzellen oder Makrophagen.

Die Promotoren sind z.B. zellunspezifisch und zellzyklusspezifisch oder spezifisch für Endothelzellen, glatte Muskelzellen oder Makrophagen und zellzyklusspezifisch.

Stukturgene für die Inhibition der Gerinnung oder für die Förderung der Fibrinolyse kodieren zum Beispiel für Tissue Plasminogen Activator (tPA), Urokinase-type Plasminogen Activator (uPA), Hybride von tPA und uPA, Protein C, Hirudin, Serin Proteinase Inhibitoren (Serpine), wie beispielsweise C-1S-Inhibitor, α1-Antitrypsin oder Antithrombin III oder Tissue Factor Pathway Inhibitor (TFPI). Strukturgene für die Förderung der Gerinnung kodieren zum Beispiel für F VIII, F IX, von Willebrand factor, F XIII, PAI-1, PAI-2 oder Tissue Factor and Fragmente hiervon. Strukturgene für Angiogenesefaktoren kodieren zum Beispiel für VEGF oder FGF. Strukturgene für die Blutdrucksenkung kodieren zum Beispiel für Kallikrein oder Endothelzell "nitric oxide synthase". Strukturgene für die Inhibition der Proliferation von glatten Muskelzellen nach Verletzungen der Endothelschicht kodieren zum Beispiel für ein antiproliferatives, zytostatisches oder zytotoxisches Protein oder ein Enzym zur Aufspaltung von Vorstufen von Zytostatika in Zytostatika, wie bereits oben unter Tumortherapie aufgeführt, oder ein Fusionsprotein eines dieser Wirkstoffe mit einem Liganden, beispielsweise einem Antikörper oder Antikörperfragmenten spezifisch für Muskelzellen. Strukturgene für weitere Blutplasmaproteine kodieren zum Beispiel für Albumin, C1-Inaktivator, Serum Cholinesterase, Transferrin oder 1-Antritrypsin.

Die Verwendung von Nukleinsäurekonstrukten für Impfungen wurde bereits in den Patentanmeldungen WO96/06941, DE19617851.7, DE19639103.2 und DE19651443.6, auf welche Bezug genommen wird, näher beschrieben. Als Zielzellen eignen sich z.B. Muskelzellen, Makrophagen und/oder Lymphozyten oder Endothelzellen. Die Promotoren sind z.B. unspezifisch und zellzyklusspezifisch oder zielzellspezifisch und zellzyklusspezifisch.

Als Strukturgene für die Prophylaxe von Infektionserkrankungen dient z.B. die DNA eines vom Infektionserreger gebildeten Proteins, welches durch Auslösung einer Immunreaktion, d.h. durch Antikörperbindung und/oder durch zytotoxische T-Lymphozyten zur Neutralisierung und/oder zur Abtötung des Erregers führt. Derartige sogenannte Neutralisationsantigene werden als Impfantigene bereits angewandt (siehe Übersicht bei Ellis, Adv. Exp. Med. Biol., 327, 263 (1992)). Die Möglichkeiten, auf konventionellem Wege wirkungsvolle Impfstoffe herzustellen, sind jedoch beschränkt. DNA-Vakzinen werfen zudem Fragen zur Wirksamkeitsstärke auf (Fynan et al., Int. J. Immunopharm., 17, 79 (1995); Donnelly et al., Immunol. 2, 20 (1994)). Ein Vorteil der vorliegenden Erfindung ist, daß mit einer größeren Wirksamkeit zu rechnen ist.

Bevorzugt im Sinne der vorliegenden Erfindung wird daher eine DNA kodierend für Neutralisationsantigene folgender Erreger: Influenza A-Virus, HIV, Tollwut-Virus, HSV (Herpes Simplex Virus), RSV (Respiratory Syncytial Virus), Parainfluenza-Virus, Rotavirus, VZV (Varizella Zoster Virus), CMV (Cytomegalo-Virus), Masern-Virus, HPV (Humanes Papillomvirus), HBV (Hepatitis B-Virus), HCV (Hepatitis C-Virus), HDV (Hepatitis D-Virus), HEV (Hepatitis E-Virus), HAV (Hepatitis A-Virus), Vibrio Cholera-Antigen, Borrelia Burgdorferi, Helicobacter pylori oder Malaria-Antigen.

Zu derartigen Wirksubstanzen gehört jedoch auch die DNA eines Antiidiotyp-Antikörpers oder seiner Antigen-bindenden Fragmente, dessen Antigenbindungsstrukturen (die "complementary determining regions") Kopien der Protein- oder Kohlenhydratstruktur des Neutralisationsantigens des Infektionserregers darstellen. Antiidiotyp-Antikörper können besonders Kohlenhydratantigene bei bakteriellen Infektionserregern ersetzen. Antiidiotypische Antikörper und ihre Spaltprodukte wurden von Hawkins et al. (J. Immunother., 14, 273 (1993)) und Westerink und Apicella (Springer Seminars in Immunopathol., 15, 227 (1993)) übersichtlich beschrieben.

Als Strukturgene für "Tumorvakzinen" gehören z.B. Gene, die für Antigene auf Tumorzellen kodieren. Derartige Antigene wurden zum Beispiel von Sedlacek et al., Contrib. to Oncol., 32, Karger Verlag, München (1988) und Contrib. to Oncol, 43, Karger Verlag, München (1992) übersichtlich dargestellt.

Weitere Beispiele stellen die Gene dar, die für folgende Antigene bzw. für folgende Antiidiotypantikörper kodieren, dar: Sialyl Lewis, Peptide auf Tumoren, welche von T-Zellen erkannt werden, von Onkogenen exprimierte Proteine, Blutgruppenantigene und deren Vorläufer, Antigene auf dem polymorphen epithelialen Mucin oder Antigene auf Heat Shock Proteinen.

Die Therapie von chronischen Infektionserkrankungen wurde bereits in den Patentanmeldungen WO96/06941, DE19617851.7, DE19639103.2 und DE19651443.6, auf welche Bezug genommen wird, näher beschrieben. Als Zielzelle eignet sich Leberzelle, Lymphozyt und/oder Makrophage, Epithelzelle oder Endothelzelle. Die Promotoren sind z.B. virusspezifisch oder zellspezifisch und zellzyklusspezifisch.

Strukturgene kodieren beispielsweise für ein Protein, welches zytostatische, apoptotische oder zytotoxische Wirkungen aufweist, oder ein Enzym, welches eine Vorstufe einer antiviralen oder zytotoxischen Substanz in die aktive Substanz spaltet. Strukturgene kodierend für antivirale Proteine sind z.B. die Gene für antiviral wirksame Cytokine und Wachstumsfaktoren, wie beispielsweise IFNα, IFNβ, IFN-γ, TNFβ, TNFα, IL-1 oder TGFβ, oder Antikörper einer Spezifität, die das jeweilige Virus inaktiviert oder dessen V_{H} und V_{L} enthaltende Fragmente oder dessen über einen Linker verbundene V_{H} und V_{L} Fragmente wie bereits beschrieben. Antikörper gegen Virusantigen sind beispielsweise: anti HBV, anti HCV, anti HSV, anti HPV, anti HIV, anti EBV, anti HTLV, Anti Coxsackie Virus oder anti Hantan Virus. Ein weiteres antivirales Protein ist z.B. ein Rev-bindendes Protein. Dieses Protein bindet an die Rev-RNA und inhibiert Rev-abhängige posttranskriptionelle Stufen der Retrovirus-Genexpression. Beispiele für Revbindende Proteine sind RBP9-27, RBP1-8U, RBP1-8D oder Pseudogene von RBP1-8.

Ein anderes virales Strukturgen kodiert für Ribozyme, welche die mRNA von Genen für Zellzykluskontrollproteine oder die mRNA von Viren verdauen. Ribozyme katalytisch für HIV wurden beispielsweise von Christoffersen et al., J. Med. Chem., 38, 2033 (1995) übersichtlich beschrieben.

Strukturgene, die für antibakterielle Proteine kodieren, sind z.B. Gene für Antikörper, die bakterielle Toxine neutralisieren oder Bakterien opsonieren. Beispielsweise gehören hierzu Antikörper gegen Meningokokken C oder B, E. coli, Borrelia, Pseudomonas, Helicobacter pylori oder Stabphylococcus aureus.

### IV) Kombination gleicher oder unterschiedlicher Strukturgene

Eine detaillierte Beschreibung erfolgte bereits in WO96/06941, WO96/06939, WO96/06940, WO96/06938, DE19639103.2, DE19651443.6, auf welche Bezug genommen wird. Ein Beispiel einer Kombination von Strukturgenen ist ein selbstverstärkendes ggf. pharmakologisch kontrollierbares Expressionssystem, in welchem eine Kombination der DNA-Sequenzen von zwei gleichen oder zwei unterschiedlichen Strukturgenen [Komponente c) und c')] vorliegt. Zur Expression beider DNA-Sequenzen ist eine weitere Promotorsequenz oder vorzugsweise die cDNA einer "internal ribosome entry site" (IRES) als regulatorisches Element zwischen beiden Stukturgenen geschaltet. Eine IRES ermöglicht die Expression zweier über eine IRES miteinander verbundener DNA-Sequenzen. Derartige IRES wurden beispielsweise von Montford und Smith (TIG, 11, 179 (1995); Kaufman et al., Nucl. Acids Res., 19, 4485 (1991); Morgan et al., Nucl. Acids Res., 20, 1293 (1992); Dirks et al., Gene, 128, 247 (1993); Pelletier und Sonenberg, Nature, 334, 320 (1988) und Sugitomo et al., BioTechn., 12, 694 (1994)) beschrieben. So kann beispielsweise die cDNA der IRES-Sequenz des Poliovirus (Position ≤ 140 bis ≥ 630 des 5' UTR verwendet werden.

Bevorzugt sind Strukturgene, die eine additive Wirkung aufweisen und über weitere Promotorsequenzen oder eine IRES-Sequenz verknüpft sind. Bevorzugte Kombinationen von Strukturgenen für die Therapie von Tumoren kodieren beispielsweise für gleiche oder unterschiedliche, zytostatische, apoptotische, zytotoxische oder entzündungserregende Proteine und/oder gleiche oder unterschiedliche Enzyme für die Spaltung der Vorstufe eines Zytostatikums; für die Therapie von Autoimmunerkrankungen für unterschiedliche Cytokine oder Rezeptoren mit synergistischer Wirkung zur Hemmung der zellulären und/oder humoralen Immunreaktion oder unterschiedliche oder gleiche TIMPs; für die Therapie von mangelhafter Bildung von Zellen des Blutes für unterschiedliche, hierarchisch aufeinanderfolgende Cytokine, wie beispielsweise IL-1, IL-3, IL-6 oder GM-CSF und Erythropoietin, G-CSF oder Thrombopoietin; für die Therapie von Nervenzellschäden für einen neuronalen Wachstumsfaktor und ein Cytokin oder einen Inhibitor eines Cytokines; für die Therapie von Störungen des Blutgerinnungs- und Blutkreislaufsystem, für ein Antithrombotikum und ein Fibrinolytikum (z.B. tPA oder uPA) oder ein zytostatisches, apoptotisches oder zytotoxisches Protein und ein Antithrombotikum oder ein Fibrinolytikum oder mehrere unterschiedliche, synergistisch wirkende Blutgerinnungsfaktoren, beispielsweise F VIII und vWF oder F VIII und F IX; für Impfungen für ein Antigen und ein immunstimulierendes Cytokin, wie beispielsweise IL-1α, IL-1β, IL-2, GM-CSF, IL-3 oder IL-4 Rezeptor, unterschiedliche Antigene eines Infektionserregers oder unterschiedlicher Infektionserreger oder unterschiedliche Antigene eines Tumortyps oder unterschiedlicher Tumortypen; für die Therapie von viralen Infektionserkrankungen für ein antivirales Protein und ein zytostatisches, apoptotisches oder zytotoxisches Protein, oder Antikörper gegen unterschiedliche Oberflächenantigene eines Virus oder mehrere Viren; und für die Therapie von bakteriellen Infektionserkrankungen für Antikörper gegen unterschiedliche Oberflächenantigene und/oder Toxine eines Keimes.

### V) Einfügung von Signalsequenzen und Transmembrandomänen

Eine detaillierte Beschreibung erfolgte bereits in den Patentanmeldungen DE19639103.2, DE19651443.6, auf welche Bezug genommen wird.

Zur Verstärkung der Translation kann am 3' Ende der Promotorsequenz und unmittelbar am 5' Ende des Startsignals (ATG) der Signal- bzw. Transmembransequenz die Nukleotidsequenz GCCACC oder GCCGCC eingefügt (Kozak, J. Cell Biol., 108, 299 (1989)) werden.

Zur Erleichterung der Sekretion des Expressionsproduktes des Strukturgenes kann die ggf. in der DNA-Sequenz des Strukturgenes enthaltende homologe Signalsequenz ersetzt werden durch eine heterologe, die extrazelluläre Ausschleusung verbessernde Signalsequenz, ersetzt werden. So kann beispielsweise die Signalsequenz für das Immunglobulin (DNA Position 63 bis 107; Riechmann et al., Nature, 332, 323 (1988)) oder die Signalsequenz für das CEA (DNA-Position 33 bis 134; Schrewe et al., Mol. Cell Biol., 10, 2738 (1990); Berling et al., Cancer Res., 50, 6534 (1990)) oder die Signalsequenz des humanen Respiratory Syncytial Virus Glycoproteins (cDNA der Aminosäuren 38 bis 50 oder 48 bis 65; Lichtenstein et al., J. Gen. Virol., 77, 109 (1996)) eingefügt werden.

Zur Verankerung des Wirkstoffes in die Zellmembran der den Wirkstoff bildenden transduzierten Zelle kann alternativ oder zusätzlich zur Signalsequenz eine Sequenz für eine Transmembrandomäne eingeführt werden. So kann beispielsweise die Transmembransequenz des menschlichen Makrophagenkolonie-stimulierenden Faktors (DNA-Position 1485 bis 1554; Cosman et al., Behring Inst. Mitt., 77, 15 (1988)) oder die DNA-Sequenz für die Signal- und Transmembranregion des menschlichen Respiratory Syncytial Virus (RSV)-Glykoproteins G (Aminosäuren 1 bis 63 oder deren Teilsequenzen, Aminosäuren 38 bis 63; Vijaya et al., Mol. Cell Biol., 8, 1709 (1988); Lichtenstein et al., J. Gen. Virol., 77, 109 (1996)) oder die DNA-Sequenz für die Signal- und Transmembranregion der Influenzavirus-Neuraminidase (Aminosäuren 7 bis 35 oder die Teilsequenz Aminosäuren 7 bis 27; Brown et al., J .Virol., 62, 3824 (1988)) zwischen der Promotorsequenz und der Sequenz des Strukturgens eingefügt werden.
Zur Verankerung des Wirkstoffes in die Zellmembran der den Wirkstoff bildenden transduzierten Zellen kann jedoch auch die Nukleotidsequenz für einen Glykophospholipid-Anker eingefügt werden. Die Einfügung eines Glykophospholipid-Ankers erfolgt am 3' Ende der Nukleotidsequenz für das Strukturgen und kann zusätzlich zur Einfügung einer Signalsequenz erfolgen. Glykophospholipid-Anker sind beispielsweise für das CEA, für das N-CAM und für weitere Membranproteine, wie beispielsweise Thy-1, beschrieben worden (siehe Übersicht Ferguson et al., Ann. Rev. Biochem., 57, 285 (1988)).

Eine weitere Möglichkeit der Verankerung von Wirkstoffen an die Zellmembran entsprechend der vorliegenden Erfindung ist die Verwendung einer DNA-Sequenz für ein Ligand-Wirkstoff-Fusionsprotein. Die Spezifität des Liganden dieses Fusionsproteins ist gerichtet gegen eine Membranstruktur auf der Zellmembran der gewählten Zielzelle.

Zu den Liganden, welche an die Oberfläche von Zellen binden, gehören beispielsweise Antikörper oder Antikörperfragmente, die gegen Strukturen auf der Oberfläche von beispielsweise Endothelzellen gerichtet sind. Insbesondere zählen hierzu Antikörper gegen die VEGF-Rezeptoren oder gegen Kinin-Rezeptoren. Sie können auch gegen Muskelzellen gerichtet sein, wie Antikörper gegen Actin oder Antikörper gegen Angiotensin II-Rezeptoren oder Antikörper gegen Rezeptoren für Wachstumsfaktoren, wie beispielsweise gegen EGF-Rezeptoren oder gegen PDGF-Rezeptoren oder gegen FGF-Rezeptoren oder Antikörper gegen Endothelin A-Rezeptoren.
Zu den Liganden gehören auch Antikörper oder deren Fragmente, welche gegen tumorspezifische oder tumorassoziierte Antigene auf der Tumorzellmembran gerichtet sind. Derartige Antikörper wurden bereits beschrieben. Die murinen monoklonalen Antikörper sind bevorzugt in humanisierter Form einzusetzen. Fab und rekombinante Fv-Fragmente und deren Fusionsprodukte werden, wie bereits beschrieben, nach den dem Fachmann bekannten Verfahren hergestellt.

Zu den Liganden gehören des weiteren alle Wirkstoffe, wie beispielsweise Cytokine oder Adhäsionsmoleküle, Wachstumsfaktoren oder deren Fragmente bzw. Teilsequenzen von ihnen, Mediatoren oder Peptidhormone, welche an Membranstrukturen oder Membranrezeptoren auf der jeweiligen ausgewählten Zelle binden. Beispielsweise gehören hierzu Liganden für Endothelzellen, wie IL-1, PDGF, bFGF, VEGF, TGGβ (Pusztain et al., J. Pathol., 169, 191 (1993)) oder Kinn und Derivate oder Analoga von Kinin. Des weiteren gehören hierzu Adhäsionsmoleküle Derartige Adhäsionsmoleküle, wie beispielsweise SLex, LFA-1, MAC-1, LeCAM-1, VLA-4 oder Vitronectin und Derivate oder Analoga von Vitronectin, wurden bereits für Endothelzellen beschrieben (Übersichten bei Augustin-Voss et al., J. Cell Biol., 119, 483 (1992); Pauli et al., Cancer Metast. Rev., 9, 175 (1990); Honn et al., Cancer Metast. Rev., 11, 353 (1992); Varner et al., Cell Adh. Commun., 3, 367 (1995)).

Die Erfindung wird an folgenden Figuren, Tabellen und Beispielen näher erläutert, ohne sich darauf zu beschränken.

### Beschreibung der Figuren und Tabellen

- Fig. 1:: Schematische Darstellung eines erfindungsgemäßen Nukleinsäurekonstrukts mit den Komponenten a) - d)
- Fig. 2:: Schematische Darstellung eines erfindungsgemäßen Nukleinsäurekonstrukts mit den Komponenten a) - e)
- Fig. 3:: Schematische Darstellung einer Aktivator-responsiven Promotoreinheit
- Fig. 4:: Schematische Darstellung eines erfindungsgemäßen Nukleinsäurekonstrukts mit einer Aktivator-responsiven Promotoreinheit
- Fig. 5:: Genomische Struktur des murinen cdc25B-Promotor/Enhancerbereiches. Aus den drei isolierten Phagenklonen wurden mittels Restriktionsverdaus verschiedener Enzyme eine Karte des genomischen Locus angefertigt. Ein unmittelbar an den 5'-Bereich der cDNA grenzendes 4,6 kb großes Fragment wurde aus dem Phagen VI ausgeschnitten und in den Bluescript SKII Vektor (Stratagene)subkloniert.
a) = Phagenklone
b) = subkloniertes Fragment
- Fig. 6:: Deletionsmutanten des murinen cdc25B Promotors. Dargestellt sind verschiedene 5'-Deletionen sowie eine 3'-Deletion des Promotors und die putativen Transkriptionsfaktor-Bindestellen, welche sich in diesem Bereich des Promotors befinden. Die Bezeichnung der einzelnen Deletionskonstrukte richtet sich nach der Lage des 5'-Endes in der Sequenz.
Die Fragmente wurden über QIAquick™ Spin Columns (Qiagen) aufgereinigt und in den pGL3 Vektor (Promega) kloniert.
- Tab. 1:: Sequenzierter Bereich des murinen cdc25B Promotors. Es wurde der Bereich sequenziert, welcher sich direkt 5' an die publizierte cDNA-Sequenz (Kakizuka et al., Genes Dev. 6, 587 (1992)) anschließt. Dargestellt sind die Anordnung der putative Bindestellen sowie der Transkriptions-Start. Bei den putativen Bindestellen handelt es sich einerseits um Aktivatoren, wie sie in vielen durch Repression regulierten zellzyklusspezifischen Promotoren vorkommen. Des weiteren finden sich putative E2F-Bindestellen und im 5'-Bereich zwei E-Boxen, denen eine repressorische Aktivität zukommt. Die dargestellte TATA-Box ist ein für zellzyklusspezifisch regulierte Gene ungewöhnliches Sequenzelement, welches in diesem Promotor offensichtlich funktionell wichtig ist, da es die Position des Transkriptionsstarts festlegt.
- Tab. 2:: Promotoraktivität der verschiedenen Deletionskonstrukte. Dargestellt ist die relative Luziferaseaktivität der in Fig. 6 dargestellten Konstrukte in wachsenden und serumdeprivierten NIH-3T3 Zellen. Die Zellzyklus-Induktion des Promotors, welche sich aus dem Quotienten der Werte für wachsende versus gehungerte Zellen ergibt, ist in der letzten Spalte angegeben. Der Wert des längsten Konstruktes in wachsenden Zellen ist dabei gleich 100 gesetzt, die restlichen Werte sind darauf abgeglichen
- Tab. 2a:: Größere Deletionen zur Bestimmung funktioneller Bereiche im Promotor und Punktmutation der TATA-Box.
- Tab. 2b:: Sequentielle Deletion der proximalen Sp1- und der NF-Y-Bindestelle und Punktmutation der NF-Y-Bindestelle. Hierbei ist die Aktivität des Konstruktes in wachsenden Zellen, welches alle diese Aktivatorbindestellen enthält, der Einfachheit halber wiederum gleich 100 gesetzt (die tatsächliche Aktivität entspricht nicht der des Konstruktes B-950).
a) geprüfte Deletionskonstrukte (s. Fig. 6)
b) Promotoraktivität in wachsenden Zellen
c) Promotoraktivität in ruhenden (serumdeprivierten) Zellen
d) Zellzyklusinduktion
   (Quotient aus Promotoraktivität in wachsenden versus ruhenden Zellen)

### Beispiele

### 1. Klonierung und Analyse des murinen cdc25B Promotors

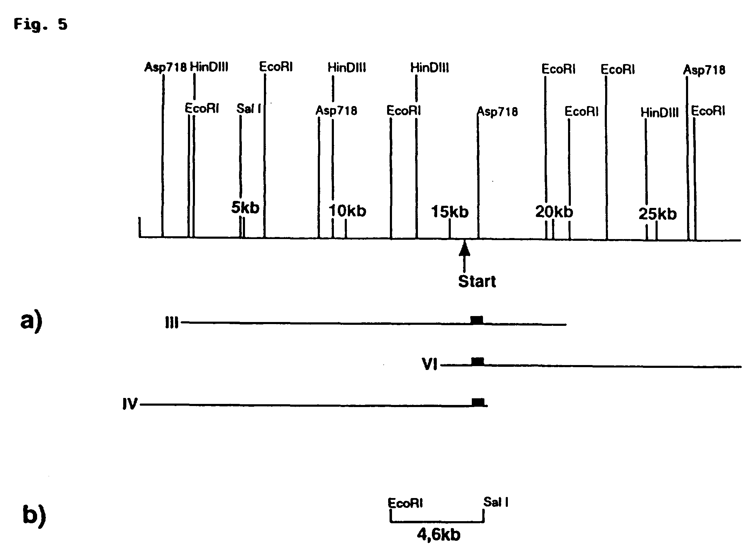

Zur Klonierung des murinen cdc25B Promotors wurden ca. 10⁶ Phagenplaques einer murinen genomischen Phagenbank (Maus-Stamm 129 FVJ, Stratagene) in A-Fix (Stratagene) gescreent. Als Sonde wurde dabei ein 80bp langes Oligonukleotid verwendet, welches gegen den äußeren 5'-Bereich der murinen cdc25B-cDNA (Kakizuka et al., Genes Dev., 6, 587 (1992)) gerichtet war. Die Sequenz lautet:
- Sonde 1:: 5'TCTAGCTAGCCTTTGCCCGCCCCGCCAC-GATGGAGGTACCCCTGCAGAAGTCTGCGCCGGGTTCAGCTCTCAGTCCTGCC-3'. (SEQ ID NO.: 4)
Drei der erhaltenen sechs Phagenklone wurden isoliert, amplifiziert und mittels Restriktionsverdaus (Enzyme: Fa. Gibco) und zweier zusätzlicher Sonden, welche sich gegen weitere 3'-gelegene Sequenzen der murinen cdc25B-cDNA richteten, kartiert (Fig. 5).
- Sonde 2:: 5'GGTCATTCAAAATGAGCAGTTACCATAAAACGCTTCCGATCC TTACCAGTGAGGCTTGCTGGAACACACTCCGGTGCTG-3' (SEQ ID NO.: 5)
- Sonde 3:: 5'GTTAAAGAAGCATTGTTATTATGGGGAGGGGGGAGCAACCTC TGGGTTCAGAATCTACATATGCTGGAAGGCCCCAATGA-3' (SEQ ID NO.: 6)

Schließlich wurde ein 4,6 kb großes Fragment aus dem proximalen, an die publizierte cDNA (Kakizuka et al. Supra) angrenzenden Enhancerbereich mit den Enzymen EcoRI und Sal I (Gibco) aus dem Phagen VI ausgeschnitten, über Agarose-Gelelektrophorese und mittels QIAquick™ Spin Columns (Qiagen) aufgereinigt und in einen Bluescript SKII Vektor (Stratagene) inseriert (Fig. 5). Von den klonierten 4,6 kb wurden 1,5 kb des 3'-Bereiches sequenziert und per Sequenzvergleich mit cdc25B-cDNA Sequenzen verschiedener Spezies als murines Homolog des cdc25B Gens identifiziert.

Aus diesem sequenzierten Bereich wurden verschiedene Fragmente herausgeschnitten, in einen pGL3 Luciferase Reporter Vektor (Promega) kloniert und in NIH-3T3 Mausfibroblasten (ATCC) auf Promotoraktivität getestet. Die (transiente) Transfektion erfolgte nach der DEAE/Dextran Methode (modifiziert nach Sompayrac et al., PNAS, 78, 7575 (1981)). Als Kontrollen dienten dabei der SV40 Basalpromotor im pGL3 Vektor (Promega), welcher keiner nennenswerten Zellzyklusregulation unterliegt, bzw. als Positivkontrolle ein Fragment des humanen cdc25C Promotors (C290, Lucibello et al., EMBO J., 14, 132 (1995)), welches ebenfalls in den pGL3 Vektor kloniert wurde. Die Luciferase-Aktivitätsbestimmung wurde durchgeführt wie beschrieben (Herber et al., Oncogene, 9, 1295 (1994)).

Die Nukleotidsequenz -950 bis + 167 erwies sich als der Promotor des murinen cdc25B-Genes (SEQ ID NO.: 7, siehe Tab. 1).

Von dem Promotor des murinen cdc25B-Genes wurden verschiedene Deletionsfragmente herausgeschnitten (Fig. 6), in einem pGL3 Luciferase-Reporter-Vektor (Promega) kloniert und in NIH-3T3-Mausfibroblasten, wie oben angeführt, auf Promotoraktivität getestet.

Zur Analyse der Zellzyklusreaktion der verschiedenen Deletionskonstrukte wurden jeweils normalwachsende, transient transfizierte mit nach der Transfektion Serumdeprivierten Zellen verglichen wie beschrieben (Lucibello et al., EMBO J., 14, 132 (1995)). Die Ergebnisse sind in Tab. 2 zusammengefaßt. Das längste Konstrukt B-950 (Nukleotidsequenz -950 bis + 167) zeigte dabei eine Zellzyklusregulation von 10,1 die vergleichbar war mit der des humanen cdc25C Konstruktes (in Tab. 2 nicht aufgeführt). Die Deletion des 3'-Bereiches bis auf + 3 (s. Abb. 6) ergab keinen Verlust von Aktivität oder Zellzyklusregulation des Promotors, so daß sich der für die Promotorregulation verantwortliche Bereich weiter eingrenzen läßt. Die Promotordeletionen des 5'-Bereiches führten zu zwei verschiedenen Effekten: zum einen führte die Deletion des längsten Konstruktes B-950 zum B-340 zu einem Anstieg der Aktivität in G₀-/G₁-Zellen, was also einer Deregulation entspricht. Weitere Deletionen bewirken ein Absinken der Promotoraktivität bis hin zur letzten Deletion, welche eine erneute Deregulation bewirkt (Tab. 2a).

Die Bestimmung der Startstelle wurde mittels Primer Extension durchgeführt. Dazu wurde RNA aus normalwachsenden NIH-3T3 Mausfibroblasten isoliert und die Reaktion mit verschiedenen Primern und MMLV-Reverser Transkriptase (Gibco) durchgeführt. Die kartierte Startstelle liegt in einem Initiator-ähnlichen Sequenzelement, 24 Basenpaare 3' der TATA-Box (SEQ ID NO.: 7, siehe Tab. 3).

Betrachtet man die Promotoraktivität der Deletionskonstrukte vor dem Hintergrund der in Tab. 3 aufgeführten putativen Transkriptionsfaktor-Bindungsstellen, so werden diese Effekte offenbar durch die Deletion bestimmter Bindestellen vermittelt: Die Deletion der im 5'-Bereich gelegenen E-Boxen führt, ebenso wie die Deletion der nahe der TATA-Box gelegenen putativen E2F-Bindestelle, zu einer Derepression des Promotors. Dahingegen bewirken die Deletionen der putativen Aktivatorbindestellen (vorwiegend SP1-Bindestellen) und eine NF-Y Bindestelle ein Absinken der Promotoraktivität (s. Tab. 2b). Die Punktmutation der putativen NF-Y site führte dabei zu einem Aktivitätsverlust von über 74% gegenüber dem Wildtyp-Konstrukt (s. Tab. 2b) Elektrophoretic Mobility Shift Assay (EMSAs, wie beschrieben in Zwicker et al., Nucleid Acids Res. 23, No. 19, S. 3822 ff., 1995) mit Hilfe spezifischer Antikörper gegen Sp1/Sp3 und NF-Y (Santa Cruz) und Kreuzkompetitionsexperimente mit boná fide Sp1 - bzw. NF-Y Bindestellen zeigten eine spezifische Bindung von Sp1/Sp3 und NF-Y an die respektiven putativen Bindestellen.

Das kürzeste Konstrukt (B-30), welches die TATA-Box und die katierte Startstelle enthält, ist zwar weitgehend dereguliert, zeigt aber eine Aktivität, die die Hintergrundaktivität des pGL3 Vektors um das Zweihundertfache übersteigt. Weiterhin führte die Punktmutation der TATA-Box zu einem Verlust der Promotoraktivität um über 25% (s. Tab. 2a), was ihre Funktionalität in der Regulation der Promotoraktivität bestätigt.

Die Transkriptionsfaktor-Bindestellen (vorwiegend SP1 und NF-Y) entsprechen denen vieler beschriebener, durch Repression oder Aktivierung zellzyklusregulierter Gene (zur Übersicht siehe Zwicker und Müller, TiGS 14, 3 (1997)), es wurde jedoch bislang noch kein Promotor eines Zellzyklusgenes beschrieben, welcher eine funktionelle TATA-Box enthielt.

Der Promotor des murinen cdc25B-Genes umfaßt somit die Nukleotide ≤ -950 bis ≥ + 167 oder Teilsequenzen dieser Nukletidsequenzen, beispielsweise ≤ - 950 bis ≥ + 1, -930 bis + 167, -720 bis + 167, -340 bis + 167, -180 bis 167, -100 bis + 167, -80 bis + 167, -60 bis + 167 oder -30 bis + 167 bzw. entsprechende Teilsequenzen bis + 3 oder + 1.

Ausgehend von den gefundenen murinen Promotorsequenzen, ist es für einen Fachmann nun ein leichtes nicht-murine cdc25B-Promotoren aufzufinden, die homolog zum murinen cdc25B-Promotor sind, indem der murine Promotor markiert, vorzugsweise radioaktiv markiert wird und mittels Hybridisierung unter stringenten Bedingungen genomische DNA-Bibliotheken, erhalten aus Saugerzellen, gescreent werden.

### 2. Herstellung von Genkonstrukten mit Hilfe der multiplen Promotortechnologie

### a) Herstellung einer Aktivator-responsiven Promotoreinheit

Die erfindungsgemäße Aktivator-responsive Promotoreinheit besteht aus folgenden unterschiedlichen, stromabwärts aufeinanderfolgenden Nukleotidsequenzen:

### Aktivatorsubeinheit A

- der Promotor des cdc25B Genes (Nukleinsäuren -950 bis + 167
- dem nuklearen Lokalisationssignal (NLS) von SV40 (SV40 Large T, Aminosäuren 126-132; PKKKRKV, Dingwall et al., TIBS, 16, 478 (1991))
- der sauren Transaktivierungsdomäne (TAD) von HSV-1 VP16 (Aminosäuren 406 bis 488; Triezenberg et al., Genes Developm., 2, 718 (1988); Triezenberg, Curr. Opin. Gen. Developm., 5, 190 (1995))
- der cDNA für den zytoplasmatischen Teil des CD4 Glycoproteins (Aminosäuren 397-435; Simpson et al., Oncogene, 4, 1141 (1989); Maddon et al., Cell , 93 (1985))

### Aktivatorsubeinheit B

- dem Promotor des cdc25C-Gens (Nukleinsäuren -290 bis + 121; Zwicker et al., EMBO J., 14, 4514 (1995); Zwicker et al., Nucl. Acids Res., 23, 3822 (1995))
- dem nuklearen Lokalisationssignal (NLS) von SV40 (SV40 Large T; Aminosäuren 126-132 PKKKRKV; Dingwall et al., TIBS, 16, 478 (1991))
- der cDNA für die DNA-Bindedomäne des Gal4 Proteins (Aminosäuren 1 bis 147, Chasman und Kornberg, Mol. Cell. Biol., 10, 2916 (1990))
- der cDNA für die CD4 Bindesequenz des p56 Ick Proteins (Aminosäuren 1-71; Shaw et al., Cell, 59, 627 (1989); Turner et al., Cell, 60, 755 (1990); Perlmutter et al., J. Cell. Biochem., 38, 117 (1988))

### Aktivator-responsive Promotoren

- 10x die Bindesequenz für Gal4-Bindeprotein mit der Nukleotidsequenz 5'-CGGACAATGTTGACCG-3' (Chasman und Kornberg, Mol. Cell. Biol. 10, 2916 (1989))
- der basale Promotor von SV40 (Nukleinsäuren 48 bis 5191; Tooze (ed). DNA Tumor Viruses (Cold Spring Harbor New York, New York, Cold Spring Harbor Laboratory) Effektorgen
- die cDNA für Luciferase (Nordeen BioTechniques, 6, 454 (1988))

### Die Funktionsweise der beschriebenen Aktivatorsequenz ist wie folgt:

Der Promotor cdc25B reguliert zellzyklusspezifisch die Transkription der kombinierten cDNAs für die Aktivierungsdomäne von VP16 und dem zytoplasmatischen Teil von CD4 (Aktivierungsuntereinheit A). Der Promotor cdc25C reguliert zellzyklusspezifisch die Transkription der kombinierten cDNAs für das DNA Bindeprotein von Gal4 und des CD4 bindenden Teiles des p56 Ick-Proteins (Aktivierungsuntereinheit B). Die Expressionsprodukte der Aktivatorsubeinheiten A und B dimerisieren durch Bindung der CD4-Domäne an die p56 Ick-Domäne. Das dimere Protein stellt einen chimären Transkriptionsfaktor für den aktivatorresponsiven Promotor (DNA-Sequenz für die Gal4-Bindedomänen/den SV40 Promotor) für die Transkription des Effektorgenes (= Luciferasegen) dar.

Die Verknüpfung der einzelnen Bestandteile des Konstruktes erfolgt über geeignete Restriktionsstellen, die über PCR-Amplifikation an den Termini der verschiedenen Elemente mitgeführt werden. Die Verknüpfung erfolgt mit Hilfe von dem Fachmann bekannten, für die Restriktionsstellen spezifischen Enzyme und DNA-Ligasen. Diese Enzyme sind käuflich zu erwerben.

Das so hergestellte Nukleotidkonstrukt wird in den pXP2 Plasmidvektor (Nordeen, BioTechniques, 6, 454 (1988)) einkloniert, der direkt oder in kolloidalen Dispersionssystemen für eine in vivo Applikation genutzt wird. Mit dem beschriebenen Plasmid werden in Kultur gehaltene 3T3 Fibroblasten mit der dem Fachmann bekannten Methode (Lucibello et al., EMBO J., 14, 132 (1995)) transfiziert und die Menge an Luciferase produziert von den Fibroblasten, wie von Herber et al. (Oncogene, 9, 1295 (1994)) und Lucibello et al. (EMBO J., 14, 132 (1995)) beschrieben, gemessen.

Zur Überprüfung der Zellzyklusspezifität werden die Fibroblasten durch Entzug von Serum über 48 Stunden in G₀/G₁ synchronisiert. Der DNA-Gehalt der Zellen wird nach Anfärbung mit Hoechst 33258 im Fluoreszenzaktivierungs-Cell Sorter bestimmt (Lucibello et al., EMBO J., 14, 132 (1995)).

### Folgende Ergebnisse werden erzielt:

In transfizierten Fibroblasten kann eine deutliche Zunahme der Luciferase im Vergleich zu nichttransfizierten Fibroblasten ermittelt werden. Proliferierende Fibroblasten (DNA > 2S) bilden deutlich mehr Luciferase als in G0/G1 synchronisierte Fibroblasten (DNA = 2 S). Somit führt die beschriebene Aktivator-responsive Promotoreinheit zu einer zellzyklusabhängigen Expression des Reportergenes Luciferase.

### b) Herstellung eines Hybridpromotors

Der erfindungsgemäße Hybridpromotor besteht aus folgenden unterschiedlichen, stromabwärts aufeinanderfolgenden Nukleotidsequenzen:
- dem Promotor des cdc25B-Genes
   (Nukleinsäuren -950 bis + 167. Die TATA-Box (Nukleinsäuren TATATAA in der Position -30 bis -23 ist mutiert zu TGTATAA)).
- der Sequenz GCCACC
   (Kodak, J. Cell Biol., 108, 229 (1989))
   der cDNA für das Signalpeptid des Immunglobulins (Nukleotidsequenz ≤ 63 bis ≥ 107; Riechmann et al., Nature 332, 323 (1988))
   der cDNA der β-Glucuronidase
   (Nukleotidsequenz ≤ 93 bis ≥ 1982; Oshima et al., PNAS USA, 84, 685 (1987))
- der Promotor des von Willebrand Faktor(vWF)-Genes
   (Nukleinsäuren -487 bis + 247; Jahroudi and Lynch, Mol. Cell Biol. 14, 999 (1994))
- dem Gen für das TATA-Box Binding Protein
   (Nukleinsäuresequenz + 1 bis + 1001, mutiert in den Nukleinsäuren 862 (A ausgetauscht gegen T) 889, 890 (GT ausgetauscht gegen AC) und 895 (C ausgetauscht gegen G) (Strubin und Struhl, Cell, 68, 721 (1992); Heard et al., EMBO J., 12, 3519 (1993))

Die Verknüpfung der einzelnen Bestandteile des Konstruktes erfolgt über geeignete Restriktionsstellen, die über PCR-Amplifikation an den Termini der verschiedenen Elemente mitgeführt werden. Die Verknüpfung erfolgt mit Hilfe von dem Fachmann bekannten, für die Restriktionsstellen spezifischen Enzymen und DNA-Ligasen.

Das so hergestellte Nukleotidkonstrukt wird in einem pUC18/19 Plasmidvektor einkloniert, der direkt oder in kolloidalen Dispersionssystemen für eine in vivo Applikation genutzt wird. Mit dem beschriebenen Plasmid werden in Kultur gehaltene menschliche Nabelschnurendothelzellen und Fibroblasten (Wi-38) mit der dem Fachmann bekannten Methode (Lucibello et al., EMBO J., 14, 132 (1995)) transfiziert und die Menge an β-Glucuronidase, produziert von den Endothelzellen, mit Hilfe von 4-Methylumbelliferyl-β-glucuronid als Substrat gemessen.

Zur Überprüfung der Zellzyklusspezifität werden Endothelzellen durch Entzug von Methionin über 48 Stunden in G₀/G₁ synchronisiert. Der DNA-Gehalt der Zellen wird nach Anfärbung mit Hoechst 33258 im Fluoreszenzaktivierungs-Cell Sorter bestimmt (Lucibello et al., EMBO J., 14, 132 (1995)).

### Folgende Ergebnisse werden erzielt:

In transfizierten Fibroblasten kann keine Zunahme der β-Glucuronidase im Vergleich zu nichttransfizierten Fibroblasten ermittelt werden. Transfizierte Endothelzellen exprimieren deutlich mehr β-Glucuronidase als nichttransfizierte Endothelzellen. Proliferierende Endothelzellen (DNA > 2S; S = einfacher Chromosomensatz) sekretieren deutlich mehr β-Glucuronidase als in G₀/G₁ synchronisierte Endothelzellen (DNA = 2S). Somit führt die beschriebene multiple Promotoreinheit zu einer zellspezifischen, zellzyklusabhängigen Expression des Strukturgenes β-Glucuronidase.

### c) Herstellung eines multiplen Promotors mit einem nuklearen Retentions-Signal (NRS) und einem nuklearen Export-Faktor (NEF)

Der erfindungsgemäße multiple Promotor besteht aus folgenden unterschiedlichen, stromabwärts aufeinanderfolgenden Nukleotidsequenzen:
- dem Promotor des cdc25B-Genes
   Nukleinsäuren -950 bis + 167)
- der Sequenz GCCACC; Seq.-ID.-Nr. 1
   (Kozak, J. Cell Biol., 108, 229 (1989))
   der cDNA für das Signalpeptid des Immunglobulins
   (Nukleotidsequenz ≤ 63 bis ≥ 107; Riechmann et al., Nature, 332, 323 (1988))
- der cDNA der β-Glucuronidase
   (Nukleotidsequenz ≤ 93 bis ≥ 1982), Oshima et al., PNA USA, 84, 685 (1987))
- der cDNA für RER von HIV-1 Virus als nukleares Retentions-Signal (NRS)
   (Nukleotidsequenz 7357 bis 7602; Ratner et al., Nature, 313, 277 (1985); Malim et al., Nature, 338, 254 (1989))
- der Promotor des von Willebrand Faktor (vWF)-Gens
   (Nukleinsäure -487 bis + 247; Jahroudi and Lynch, Mol. Cell Biol., 14, 999 (1994))
- der cDNA für REV von HIV-1 Virus als nuklearer Export-Faktor (NEF) (Aminosäuresequenz 1-117; Ratner et al., Nature, 313, 277 (1985))

Die Verknüpfung der einzelnen Bestandteile des Konstruktes erfolgt über geeignete Restriktionstellen, die über PCR-Amplifikation an den Termini der verschiedenen Elemente mitgeführt werden. Die Verknüpfung erfolgt mit Hilfe von dem Fachmann bekannten, für die Restriktionstellen spezifischen Enzymen und DNA-Ligasen. Diese Enzyme sind käuflich zu erwerben. Das so hergestellte Nukleotidkonstrukt wird in einem pUC18/19 Plasmidvektor einkloniert, der direkt oder in kolliodalen Dispersionssystemen für eine in vivo Applikation genutzt wird. Mit dem beschriebenen Plasmid werden in Kultur gehaltene menschliche Nabelschnurendothelzellen und Fibroblasten (Wi-38) mit der dem Fachmann bekannten Methode (Lucibello et al., EMBO J., 14, 132 (1995)) transfiziert und die Menge an β-Glucuronidase, produziert von den Endothelzellen, mit Hilfe von 4-Methylumbelliferyl-β-glucuronid als Substrat gemessen.

Zur Überprüfung der Zellzyklusspezifität werden Endothelzellen durch Entzug von Methionin über 48 Stunden in G₀/G₁ synchronisiert. Der DNA-Gehalt der Zellen wird nach Anfärbung mit Hoechst 33258 im Fluoreszensaktivierungs-Cell Sorter bestimmt (Lucibello et al., EMBO J., 14, 132 (1995)).

### Folgende Ergebnisse werden erzielt:

In transfizierten Fibroblasten kann keine Zunahme der β-Glucuronidase im Vergleich zu nichttransfizierten Fibroblasten ermittelt werden. Transfizierte Endothelzellen exprimieren deutlich mehr β-Glucuronidase als nichttransfizierte Endothelzellen. Proliferierende Endothelzellen (DNA > 2S; S = einfacher Chromosomensatz) sekretieren deutlich mehr β-Glucuronidase als in G₀/G₁ synchronisierte Endothelzellen (DNA = 2S). Somit führt die beschriebene multiple Promotoreinheit zu einer zellspezifischen, zellzyklusabhängigen Expression des Strukturgenes β-Glucuronidase.

### 3. Anwendung

Ein Wirkstoff gemäß den beschriebenen Beispielen ermöglicht nach lokaler Applikation beispielsweise an dem Ort des Tumors oder nach intrakranieller bzw. subarachnoidaler Gabe oder systemischer, bevorzugt intravenöser oder intraarterieller Gabe, daß durch die Zellzyklus- und Endothelzellspezifität der multiplen Promotoreinheit vorwiegend, wenn nicht ausschließlich, nur proliferierende Endothelzellen β-Glucuronidase ausscheiden. Diese β-Glucuronidase spaltet ein nunmehr injiziertes, gut verträgliches Doxorubicin-β-glucuronid (Jacquesy et al., EPO 0 511 917 A1) in das zytostatisch wirkende Doxorubicin. Dieses hemmt die Endothelzellproliferation und wirkt zytostatisch auf diese Zellen wie auch auf benachbarte Tumorzellen. Hierdurch kommt es zur Hemmung des Tumorwachstums.

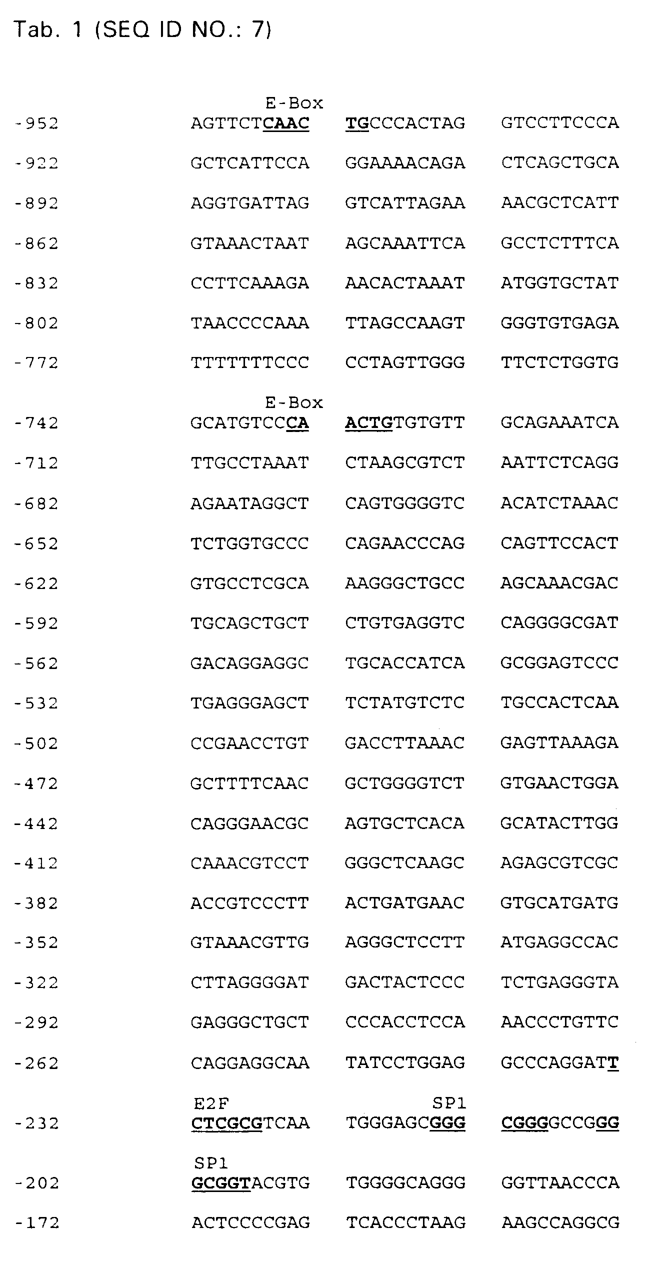

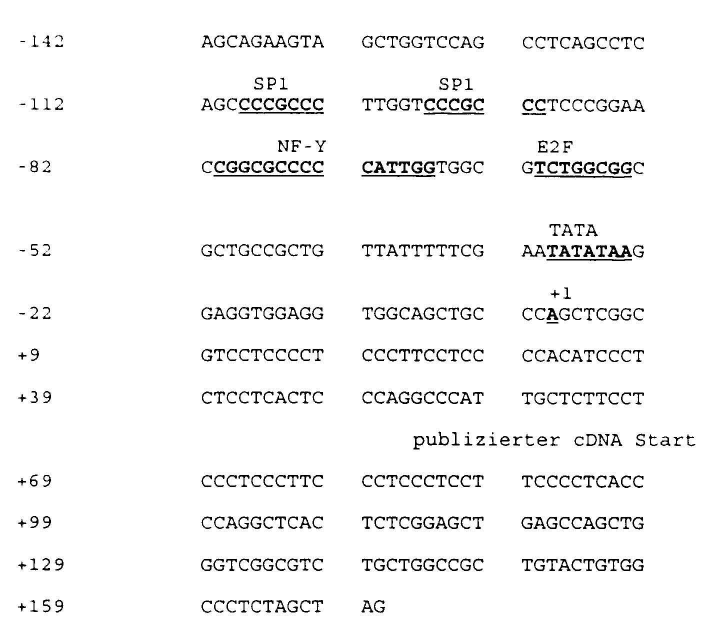

**Tab. 2a**

| a Konstrukt | b wachsende Zellen | c serumdeprivierte Zellen | d Zellzyklus-Induktion |
|---|---|---|---|
| B-950 | 100 | 9,9 | 10,1 |
| B-340 | 153,8 | 25,1 | 6,1 |
| B-180 | 121,3 | 18,0 | 6,8 |
| B-100 | 76,7 | 10,1 | 6,8 |
| B-80 | 29,9 | 8,5 | 3,5 |
| B-20 | 22,5 | 15,7 | 1,4 |
| B-950 m TATA | 72,5 | 9,0 | 7,7 |
| m = mutiert | | | |

**Tab. 2b**

| a) | b) | c) | d) |
|---|---|---|---|
| B-223 | 100 | 11,4 | 8,8 |
| B-209 | 87,5 | 11,7 | 7,5 |
| B-180 | 58,8 | 10,6 | 5,5 |
| B-100 | 28,0 | 5,0 | 5,6 |
| B-87 | 25,3 | 6,0 | 4,2 |
| B-67 | 22,0 | 8,4 | 2,6 |
| B-223mY | 26,6 | 7,0 | 3,8 |

## Patentansprüche

1. Promotor des cdc25B-Genes enthaltend eine Sequenz, die mit einer Sequenz gemäß Tabelle 1 (SEQ ID No: 7) oder einem funktionellen Teil davon unter stringenten Bedingungen hybridisiert.

2. Promotor nach Anspruch 1, dadurch gekennzeichnet, daß der Promotor die Sequenz gemäß Tabelle 1 (SEQ ID NO: 7) oder einen funktionellen Teil davon enthält.

3. Promotor nach Anspruch 1 oder 2, dadurch gekennzeichnet, daß der funktionelle Teil die TATA-Box, mindestens eine Sp1-Bindestelle und mindestens eine NFY-Bindestelle und gegebenenfalls mindestens eine E2F-Bindestelle sowie gegebenenfalls mindestens eine E-Box enthält.

4. Promotor nach einem der Ansprüche 1-3, dadurch gekennzeichnet, daß er die Sequenz umfassend die Nukleotide von ca. -950 bis ca. + 167 der Tabelle 1 enthält oder Fragmente davon, die noch alle funktionellen cis-regulatorischen Elemente der Promotorsequenz der Nukleotide von ca. -950 bis ca. + 167 gemäß Figur 6 enthalten.

5. Promotor nach einem der Ansprüche 1-3, dadurch gekennzeichnet, daß er die Sequenz umfassend die Nukleotide von ca. -950 bis ca. + 3 der Tabelle 1 enthält oder Fragmente davon, die noch alle funktionellen cis-regulatorischen Elemente der Promotorsequenz der Nukleotide von ca. -950 bis + 3 gemäß Figur 6 enthalten.

6. Promotor nach einem der Ansprüche 1-3, dadurch gekennzeichnet, daß er die Sequenz umfassend die Nukleotide von ca. -930 bis ca. + 3 der Tabelle 1 enthält oder Fragmente davon, die noch alle funktionellen cis-regulatorischen Elemente der Promotorsequenz der Nukleotide von ca. -930 bis ca. + 3 gemäß Figur 6 enthalten.

7. Promotor nach einem der Ansprüche 1-3, dadurch gekennzeichnet, daß er die Sequenz umfassend die Nukleotide von ca. -720 bis ca. + 3 der Tabelle 1 enthält oder Fragmente davon, die noch alle funktionellen cis-regulatorischen Elemente der Promotorsequenz der Nukleotide von ca. -720 bis ca. + 3 gemäß Figur 6 enthalten.

8. Promotor nach einem der Ansprüche 1-3, dadurch gekennzeichnet, daß er die Sequenz umfassend die Nukleotide von ca. -340 bis ca. 3 der Tabelle 1 enthält oder Fragmente davon, die noch alle funktionellen cis-regulatorischen Elemente der Promotorsequenz der Nukleotide von ca. -340 bis ca. + 3 gemäß Figur 6 enthalten.

9. Promotor nach einem der Ansprüche 1-3, dadurch gekennzeichnet, daß er die Sequenz umfassend die Nukleotide von ca. - 180 bis ca. + 3 der Tabelle 1 enthält oder Fragmente davon, die noch alle funktionellen cis-regulatorischen Elemente der Promotorsequenz der Nukleotide von ca. -180 bis ca. + 3 gemäß Figur 6 enthalten.

10. Promotor nach einem der Ansprüche 1-3, dadurch gekennzeichnet, daß er die Sequenz umfassend die Nukleotide von ca. - 100 bis ca. + 3 der Tabelle 1 enthält oder Fragmente davon, die noch alle funktionellen cis-regulatorischen Elemente der Promotorsequenz der Nukleotide von ca. -100 bis ca. + 3 gemäß Figur 6 enthalten.

11. Promotor nach einem der Ansprüche 1-3, dadurch gekennzeichnet, daß er die Sequenz umfassend die Nukleotide von ca. -80 bis ca. + 3 der Tabelle 1 enthält oder Fragmente davon, die noch alle funktionellen cis-regulatorischen Elemente der Promotorsequenz der Nukleotide von ca. -80 bis ca. + 3 gemäß Figur 6 enthalten.

12. Promotor nach einem der Ansprüche 1-3, dadurch gekennzeichnet, daß er die Sequenz umfassend die Nukleotide von ca. -60 bis ca. + 3 der Tabelle 1 enthält oder Fragmente davon, die noch alle funktionellen cis-regulatorischen Elemente der Promotorsequenz der Nukleotide von ca. -60 bis ca. + 3 gemäß Figur 6 enthalten.

13. Promotor nach einem der Ansprüche 1-3, dadurch gekennzeichnet, daß er die Sequenz umfassend die Nukleotide von ca. -30 bis ca. + 3 der Tabelle 1 enthält oder Fragmente davon, die noch alle funktionellen cis-regulatorischen Elemente der Promotorsequenz der Nukleotide von ca. -30 bis ca. + 3 gemäß Figur 6 enthalten.

14. Verfahren zum Auffinden von cdc25B-Promotoren, dadurch gekennzeichnet, daß ein Promotor gemäß einem oder mehreren der Ansprüche 1 bis 13 markiert, vorzugsweise radioaktiv markiert wird, und mittels Hybridisierung unter stringenten Bedingungen genomische DNA-Bibliotheken, vorzugsweise aus Säugerzellen, durchsucht werden.

15. Verfahren zur Isolierung des murinen cdc25B-Promotors, dadurch gekennzeichnet, daß eine murine genomische Phagenbank, erhalten aus dem Maus-Stamm 129FVJ, mit einer Sonde enthaltend einen Teil der Sequenz gemäß Tabelee 1 (SEQ ID NO: 7), vorzugsweise enthaltend die Sequenz SEQ ID NO.: 4, gescreent wird.

16. Nukleinsäurekonstrukt enthaltend mindestens einen Promotor gemäß einem der Ansprüche 1 bis 13.

17. Nukleinsäurekonstrukt nach Anspruch 16, dadurch gekennzeichnet, daß es zusätzlich ein Strukturgen enthält.

18. Nukleinsäurekonstrukt nach Anspruch 16, dadurch gekennzeichnet, daß der genannte Promotor stromaufwärts von dem Strukturgen angeordnet ist.

19. Nukleinsäurekonstrukt nach Anspruch 17 oder 18, dadurch gekennzeichnet, daß die 5'- nicht kodierende Region des cdc25B Genes mit den Nukleotidsequenzen von + 1 bis ca. + 167 zwischen dem genannten Promotor und dem Strukturgen eingefügt ist.

20. Nukleinsäurekonstrukt nach einem der Ansprüche 16-19, dadurch gekennzeichnet, daß der Promotor gemäß einem der Ansprüche 1-13 mit mindestens einer weiteren Aktivierungssequenz kombiniert ist, wobei diese weitere Aktivierungssequenz ausgewählt ist aus einer nichtspezifischen, virusspezifischen, metabolischspezifischen, zellspezifischen, zellzyklusspezifischen und/oder zellproliferationsabhängigen Aktivierungssequenz.

21. Nukleinsäurekonstrukt nach Anspruch 20, dadurch gekennzeichnet, daß die weitere Aktivierungssequenz ausgewählt ist aus Promotoren, die in Endothelzellen, Peritonealzellen, Pleuralzellen, Epithelzellen der Haut, Zellen der Lunge, Zellen des Gastrointestinaltraktes, Zellen der Niere und harnableitenden Wege, Muskelzellen, Bindegewebszellen, blutbildenden Zellen, Makrophagen, Lymphozyten, Leukämiezellen, Tumorzellen oder Gliazellen aktiviert werden; Promotorsequenzen von Viren wie HBV, HCV, HSV, HPV, EBV, HTLV, CMV oder HIV; Promotor- oder Enhancersequenzen, die durch Hypoxie aktiviert werden; zellzyklusspezifische Aktivierungssequenzen der Gene kodierend für cdc25C, Cyclin A, cdc2, E2F-1, B-myb und DHFR, und/oder Bindesequenzen für zellproliferationsabhängig auftretende oder aktivierte Transkriptionsfaktoren wie Monomere oder Multimere der Myc E-Box.

22. Nukleinsäurekonstrukt nach einem der Ansprüche 16-21, dadurch gekennzeichnet, daß der Promotor gemäß einem der Ansprüche 1-13 in einer Form vorhanden ist, bei der mindestens eine Bindestelle für einen Transkriptionsfaktor mutiert ist.

23. Nukleinsäurekonstrukt nach Anspruch 22, dadurch gekennzeichnet, daß die TATA-Box mutiert ist.

24. Nukleinsäurekonstrukt nach Anspruch 22 oder 23, dadurch gekennzeichnet, daß zusätzlich ein Strukturgen, eine weitere unspezifisch, zellspezifisch, virusspezifisch, durch Tetrazyklin und/oder zellzyklusspezifisch aktivierbare Promotor- oder Enhancersequenz, welche die Transkription mindestens eines weiteren Strukturgens aktiviert, das für mindestens einen Transkriptionsfaktor kodiert, welcher derart mutiert ist, daß er an die mutierte(n) Bindestelle(n) des Promotors gemäß Anspruch 22 oder 23 bindet und diesen aktiviert, und/oder das für einen Transkriptionsfaktor kodierende Strukturgen enthalten ist.

25. Nukleinsäurekonstrukt nach einem der Ansprüche 22-24, dadurch gekennzeichnet, daß der Transkriptionsfaktor ein mutiertes TATA-Box bindendes Protein (TBP) ist.

26. Nukleinsäurekonstrukt nach Anspruch 25, enthaltend
(1) den Promotor gemäß einem der Ansprüche 1-13, einschließlich TATA-Box, wobei die Sequenz der TATA-Box mutiert ist zu TGTA,
(2) die Sequenz GCCACC,
(3) die cDNA des Signalpeptids des Immunglobulin (Nukleotidsequenz ≤ 63 ≥ 107),
(4) die cDNA der β-Glucuronidase (Nukleotidsequenz ≤ 93 bis ≥ 1982),
(5) den Promotor des vWF-Genes (Nukleotidsequenz -487 bis + 247), und
(6) die cDNA des TATA-Box bindenden Proteins (Nukleinsäuresequenz von 1 bis 1001, welche an der Nukleinsäurepositionen 862 (A ausgetauscht gegen T), 889 und 890 (GT ausgetauscht gegen AC) und 895 (C ausgetauscht gegen G) mutiert ist.

27. Nukleinsäurekonstrukt, welches einen Promotor und ein Strukturgen, an dessen 3'-Ende ein nukleares Retentions-Signal (NRS) angefügt ist, und einen weiteren Promotor, welcher die Transkription des Genes kodierend für einen nuklearen Export-Faktor (NEF) aktiviert, wobei dieser NEF an die mRNA des NRS bindet, enthält, dadurch gekennzeichnet, daß mindestens einer der genannten Promotoren ein Promotor gemäß einem der Ansprüche 1-13 ist.

28. Nukleinsäurekonstrukt nach einem der Ansprüche 20-27, dadurch gekennzeichnet, daß mindestens ein Promotor bzw. Enhancer durch eine Aktivator-responsive Promotoreinheit ersetzt ist.

29. Nukleinsäurekonstrukt nach Anspruch 28, dadurch gekennzeichnet, daß die Aktivator-responsive Promotoreinheit mindestens folgende Komponenten enthält:
(1) eine oder mehrere, gleiche oder unterschiedliche Aktivatorsubeinheiten, welche von einem Promotor bzw. Enhancer in ihrer basalen Transkription aktiviert wird/werden, und
(2) einen Aktivator-responsiven Promotor, der durch das Expressionsprodukt der genannten Aktivatorsubeinheit aktiviert wird.

30. Nukleinsäurekonstrukt nach Anspruch 28 oder 29, enthaltend als eine Aktivatorsubeinheit (A),
(1) den Promotor gemäß einem der Ansprüche 1-13,
(2) das nukleare Lokalisationssignal (NLS) von SV40 (SV40 Large T, Aminosäuren 126-132; PKKKRKV),
(3) die sauren Transaktivierungsdomäne (TAD) von HSV-1 VP16 (Aminosäuren 406 bis 488), und
(4) die cDNA kodierend für den zytoplasmatischen Teil des CD4 Glycoproteins (Aminosäuren 397-435);
und als eine andere Aktivatorsubeinheit (B),
(1) den Promotor des cdc25C-Gens (Nukleinsäuren -290 bis + 121),
(2) das nukleare Lokalisationssignal (NLS) von SV40 (SV40 Large T; Aminosäuren 126-132 PKKKRKV),
(3) die cDNA für die DNA-Bindedomäne des Gal4 Proteins (Aminosäuren 1 bis 147), und
(4) die cDNA für die CD4 Bindesequenz des p56 Ick Proteins (Aminosäuren 1-71)
sowie den Aktivator-responsive Promotor, mit bis zu ca. 10 Kopien der Bindesequenz für Gal4-Bindeprotein mit der Nukleotidsequenz 5'-CGGACAATGTTGACCG-3' und dem basalen Promotor von SV40 (Nukleotidsequenz 48 bis 5191);
und gegebenenfalls ein Strukturgen, vorzugsweise eine komplette cDNA kodierend für einen Wirkstoff, ein Enzym oder ein Fusionsprotein aus einem Liganden und einem Wirkstoff oder einem Liganden und einem Enzym.

31. Nukleinsäurekonstrukt nach einem der Ansprüche 17 bis 30, dadurch gekennzeichnet, daß es sich bei dem Strukturgen um ein Gen handelt, das für einen Wirkstoff kodiert, der ausgewählt ist aus Enzyme, Fusionsproteine, Cytokine, Chemokine, Wachstumsfaktoren, Rezeptoren für Cytokine, Rezeptoren für Chemokine, Rezeptoren für Wachstumsfaktoren, antiproliferativ oder zytostatisch oder apoptotisch wirkende Peptide oder Proteine, Antikörper, Antikörperfragmente, Angiogeneseinhibitoren, Peptidhormone, Gerinnungsfaktoren, Gerinnungshemmer, fibrinolytische Proteine, auf den Blutkreislauf wirksame Peptide oder Proteine, Blutplasmaproteine, Antigene von Infektionserregern, Antigene von Zellen, Antigene von Tumoren, Thrombose induzierende Substanzen, Komplement-aktivierende Proteine, Virushüllproteine und/oder Ribozyme.

32. Nukleinsäurekonstrukt nach Anspruch 31, dadurch gekennzeichnet, daß es sich bei dem Strukturgen um ein Gen handelt, das für ein Enzym kodiert, welches eine Vorstufe eines Pharmakons in ein Pharmakon spaltet.

33. Nukleinsäurekonstrukt nach Anspruch 31 bis 32, dadurch gekennzeichnet, daß es sich bei dem Strukturgen um ein Gen handelt, das für ein Ligand-Wirkstoff-Fusionsprotein oder ein Ligand-Enzym-Fusionsprotein kodiert, wobei der Ligand ausgewählt ist aus Cytokine, Wachstumsfaktoren, Antikörper, Antikörperfragmente, Peptidhormone, Mediatoren und/oder Zelladhäsionsproteine.

34. Nukleinsäurekonstrukt nach einem der Ansprüche 16-33, dadurch gekennzeichnet, daß es sich bei der Nukleinsäure um DNS handelt.

35. Nukleinsäurekonstrukt nach einem der Ansprüche 16-34, dadurch gekennzeichnet, daß das Nukleinsäurekonstrukt in einen Vektor, vorzugsweise einen Plasmidvektor oder viralen Vektor eingefügt ist.

36. Verfahren zur Herstellung eines Nukleinsäurekonstruktes nach einem der Ansprüche 16 bis 35, dadurch gekennzeichnet, daß die einzelnen Komponenten miteinander verbunden werden.

37. Zelle, dadurch gekennzeichnet, daß sie ein Nukleinsäurekonstrukt gemäß einem der Ansprüche 16-35 enthält.

38. Verwendung eines Nukleinsäurekonstruktes nach einem der Ansprüche 16-35 oder einer Zelle nach Anspruch 37 zur Herstellung eines Arzneimittels zur Behandlung einer Erkrankung ausgewählt aus Tumorerkrankungen, Leukämien, Autoimmunerkrankungen, Allergien, Arthritiden, Entzündungen, Organabstoßungen, Transplantat gegen Wirt-Reaktionen, Blutgerinnungserkrankungen, Kreislauferkrankungen, Blutarmut, Infektionen, Hormonerkrankungen und/oder ZNS-Schäden.

39. Verwendung einer Zelle nach Anspruch 38, dadurch gekennzeichnet, daß die Zelle eine Endothelzelle ist.
